# FASCICULE DE BREVET EUROPEEN

(11) **EP 2 981 590 B1**
(45) Date de publication et mention de la délivrance du brevet: **18.09.2019**
(21) Numéro de dépôt: 14720188.3
(22) Date de dépôt: 03.04.2014
(51) Int. Cl.: C09K 9/02, A61K 49/00, C07D 257/02, C07F 5/00, C07D 213/24, G01N 21/64, G01N 33/533, G01N 33/58, C07D 213/79, C07D 401/04, C07D 255/02, C09K 11/06, C07D 401/14

(54) **COMPLEXES DE LANTHANIDE COMPRENANT AU MOINS DEUX GROUPES BETAÏNES, UTILES COMME MARQUEURS LUMINESCENTS**
LANTHANIDKOMPLEXE MIT MINDESTENS ZWEI ALS LUMINESZENZMARKER VERWENDBAREN BETAINGRUPPEN
LANTHANIDE COMPLEXES COMPRISING AT LEAST TWO BETAINE GROUPS, WHICH CAN BE USED AS LUMINESCENT MARKERS

(30) Priorité: 04.04.2013 FR 1353037
(43) Date de publication de la demande: 10.02.2016
(73) Titulaire: Ecole Normale Supérieure de Lyon, 69342 Lyon Cedex 07 (FR); Université Claude Bernard Lyon I, 69622 Villeurbanne Cedex (FR); Centre National de la Recherche Scientifique (C.N.R.S.), 75794 Paris Cedex 16 (FR)
(72) Inventeur: MAURY, Olivier, F-69126 Brindas (FR); ANDRAUD, Chantal, F-69740 Genas (FR); PLACIDE, Virginie, F-45100 Orléans (FR); PITRAT, Delphine, F-38890 Saint Chef (FR)
(74) Mandataire: Be IP
(86) Numéro de dépôt international: PCT/FR2014/050813
(87) Numéro de publication internationale: WO 2014/162105

(56) Documents cités:
- WO-A1-2009/010580
- WO-A1-2013/026790
- US-A1- 2003 138 876
- HE WEI ET AL: "Compact Zwitterion-Coated Iron Oxide Nanoparticles for Biological Applications", NANO LETTERS, vol. 12, no. 1, 11 janvier 2012 (2012-01-11), pages 22-25, XP055063107, ISSN: 1530-6984, DOI: 10.1021/nl202721q cité dans la demande
- ELEONORA MURO ET AL: "Small and Stable Sulfobetaine Zwitterionic Quantum Dots for Functional Live-Cell Imaging", JOURNAL OF THE AMERICAN CHEMICAL SOCIETY, vol. 132, no. 13, 7 avril 2010 (2010-04-07), pages 4556-4557, XP055000735, ISSN: 0002-7863, DOI: 10.1021/ja1005493 cité dans la demande
- CHANTAL ANDRAUD ET AL: "Lanthanide Complexes for Nonlinear Optics: From Fundamental Aspects to Applications", EUROPEAN JOURNAL OF INORGANIC CHEMISTRY, vol. 2009, no. 29-30, 1 octobre 2009 (2009-10-01), pages 4357-4371, XP055091656, ISSN: 1434-1948, DOI: 10.1002/ejic.200900534

## Description

La présente invention concerne le domaine technique des complexes de lanthanide. En particulier, la présente invention concerne des complexes de lanthanide comprenant au moins deux groupes bétaïnes sur leur partie organique, leur conférant des propriétés intéressantes en termes de solubilité dans l'eau et dans les milieux biologiques, et permettant de limiter, voire supprimer les phénomènes d'adhésion non spécifique avec les biomolécules (protéines...) ou les parties lipophiles des cellules, autorisant notamment leur utilisation dans des applications biologiques.

Certains complexes de lanthanide présentent des propriétés spectroscopiques remarquables (bandes d'émission fines caractéristiques d'un métal donné et à durée de vie longue) et sont, de ce fait, des composés à très fort potentiel pour des applications en imagerie biologique (S. V. Eliseeva, J.-C. G. Bünzli, Chem. Soc. Rev. 2010, 39, 189). Ces composés luminescents peuvent être utilisés seuls pour des applications en imagerie par microscopie de fluorescence mono- ou biphotonique, ou bien en conjonction avec un fluorophore approprié pour réaliser des expériences de FRET (Förster/Fluorescence Resonant Energy Transfer). Dans ce dernier cas, les complexes de lanthanide sont généralement sous la forme d'un complexe conjugué à une biomolécule. Ces deux techniques peuvent éventuellement être résolues en temps, grâce à la durée de vie longue des lanthanides, ce qui constitue un avantage important pour améliorer la détection en s'affranchissant de signaux de fluorescence parasites à durée de vie courte (C. P. Montgomery B. S. Murray, E. J. New, R. Pal, D. Parker, Acc. Chem. Res. 2009, 42, 925. E. G. Moore, A. P. S. Samuel, K. N. Raymond, Acc. Chem. Res. 2009, 42, 542.). De très nombreux composés de lanthanide luminescents ont été décrits dans la littérature pour de telles applications (M. Latva, H. Takalo, V.-M. Mukkala, C. Matachescu, J.C. Rodríguez-Ubis, J. Kankare, J. Lumin., 1997 75 149-169 ; A. Picot, A. D'Aléo, P. L. Baldeck, A. Grichine, A. Duperray, C. Andraud, O. Maury, J. Am. Chem. Soc. 2008, 130, 1532) et certains sont commercialisés comme des dérivées du DTPA (diéthylène triamine penta acide, US 5,622,821), des composés à base de pyridine ou poly-pyridine (US 4,920, 195 ; US 4,761,481 ; US 5,216,134 ; US 4,859 , 777 ; US 5,202,423 ; US 5,324,825), ou encore des cryptates macrocycliques (EP 0 180 492 ; EP 0 321353 ; EP 0 601 113 ; WO 2001/96877 ; WO 2008/063721).

US 2003/138876 décrit un agent chélatant de lanthanides comprenant des fonctions acides et des fonctions basiques. WO 2013/026790 décrit des complexes chargés de lanthanides.

Par le passé, les complexes de lanthanide avaient une structure simple avec peu de carbone, étaient généralement chargés, et de ce fait étaient donc solubles dans l'eau. Avec les développements de ces dernières années, des complexes fonctionnalisés ont été développés pour optimiser leurs propriétés spectroscopiques, déplacer leur absorption vers le visible et optimiser leur absorption biphotonique. De plus en plus, des antennes fonctionnelles généralement conjuguées et/ou aromatiques comportant un grand nombre de carbone sont introduites, ce qui conduit à des composés lipophiles, donc non solubles dans l'eau (C. Andraud, O. Maury Eur. J. Inorg. Chem. 2009, 4357-4371).

Pour augmenter l'hydrosolubilisation des complexes de lanthanide, différentes approches ont été développées. Une approche consiste à introduire des groupements polyéthylène glycol (PEG) en tant que groupements hydrosolubilisants. A. Picot, A. D'Aléo, P.L. Baldeck, A. Grichine, A. Duperray, C. Andraud et O. Maury, dans J. Am. Chem. Soc., 2008, 130, 1532-153,3 utilisent cette approche dans le cas de complexes de lanthanide dont la complexation est assurée par trois ligands intégrant un groupe 2,6-pyridine-diacide carboxylique. L'introduction de groupements PEG est également envisagée dans C. R. Chim., 2010, 13, 681-690, Inorg. Chem., 2011, 50, 4987-4999 et Angew. Chem. In. Ed. 2012, 51, 6622 -6625.

La demande de brevet WO 2013/011236, relative à des composés de lanthanide fortement luminescents, dont certains sont basés sur un cycle 1,4,7-triazacyclononane (TACN) substitué par des chromophores à base de pyridine conjuguée, utilise également des groupements PEG. Les complexes décrits sont très stables dans l'eau et la présence de groupements PEG assure leur bonne solubilité.

Ces ligands macrocycliques fonctionnels conduisent notamment à la formation de complexes d'europium qui présentent une brillance très élevée à 337 nm (la brillance est définie par le produit du rendement quantique par l'absorption à la longueur d'onde d'intérêt, ici 337nm), 337 nm correspondant à la longueur d'onde d'excitation du laser azote utilisé pour les applications commerciales (J. W. Walton, A. Bourdolle, S.J. Butler, M. Soulier, M. Delbianco, B.K. McMahon, R. Pal, H. Puschmann, , J.M. Zwier, L. Lamarque, O. Maury, C. Andraud and D. Parker Chem. Commun. 2013, 49, 1600-1602).

Ces composés peuvent également être pourvus d'une fonction réactive, pour être éventuellement conjugués par liaison covalente à une molécule d'intérêt, telle qu'une biomolécule. Dans ce cas, les composés ont pour finalité d'être liés de manière covalente à une biomolécule, en vue de réaliser des expériences de FRET (applications HTRF® commercialisées par CISBIO BIOASSAYS). Le document WO2013/026790 D2 concerne les complexes luminescents de lanthanide offrant une brillance élevée. Il s'intéresse à résoudre le problème de la solubilité de tels complexes tout en limitant l'absorption non spécifique sur les biomolécules.

Cependant, ces composés présentent deux inconvénients majeurs :
1) dans le cadre de leurs recherches, les inventeurs ont observé que ces complexes s'adsorbent de manière non spécifique sur les biomolécules, ce qui complique la préparation de biomolécules conjuguées. Ce phénomène se retrouve également dans les expériences d'imagerie par microscopie mono ou bi-photonique réalisées sur des cellules fixées. Dans ce cas, une accumulation spécifique de ces composés dans les membranes et/ou organelles lipophiles est constatée. Les inventeurs de la présente invention ont associé cet inconvénient à la présence de groupements PEG.
2) De plus, ces composés sont préparés par une voie de synthèse convergente donc peu versatile, longue et d'efficacité modeste.

D'autres solutions proposent de solubiliser les complexes de lanthanides par introduction de groupements chargés anioniques. Dans Inorg, Chem, 2009, 48(9), 4207-4218, l'hydrosolubilisation est assurée par des groupements -SO₃⁻, alors que dans Inorg, Chem., 2009, 48, 4601-4603, l'hydrosolubilisation est assurée par des groupements -CO₃⁻. Dans ces exemples, l'introduction de groupements anioniques permet d'assurer une bonne hydrosolubilisation, le problème de l'adsorption non spécifique n'est pas abordé et aucun exemple ne décrit leur comportement en présence de bioconjugués.

La présente invention se propose de fournir de nouveaux complexes de lanthanide fluorescents qui présentent une solubilité satisfaisante dans l'eau et donc en milieux physiologiques et biologiques, mais qui ne présentent pas l'inconvénient des solutions antérieures, et notamment de celles utilisant des groupements PEG, pour lesquels les inventeurs ont constaté une tendance à adhérer aux biomolécules, aux membranes cellulaires et, plus généralement, aux parties hydrophobes présentes en grand nombre dans les biomolécules et milieux biologiques.

Dans ce contexte, l'invention concerne des complexes de lanthanide luminescents comportant un agent chélatant, formé d'un macrocycle ou d'un ensemble de ligands, complexant un ion lanthanide Ln³⁺, caractérisés en ce que l'agent chélatant est substitué par au moins deux groupes bétaïne.

A titre d'exemple d'Ion lanthanide Ln³⁺, on peut citer Eu³⁺, Sm³⁺, Tb³⁺ ou Dy³⁺.

Dans le cadre de l'invention, la solubilisation des complexes de lanthanide en milieu aqueux est assurée par des groupes hydrosolubilisants zwitterioniques de type bétaïne.

La présente invention propose une fonctionnalisation de complexes de lanthanide avec deux groupes bétaïne ou plus. Cette fonctionnalisation permet de solubiliser les complexes de lanthanide dans l'eau et les milieux biologiques et permet d'éviter les phénomènes d'adhésion non spécifiques avec des biomolécules et les organelles. Cette fonctionnalisation peut être appliquée à tous types de complexes de lanthanide. De plus, dans le cadre de l'invention, il a été démontré que la présence de groupes bétaïne n'affectait pas les propriétés de luminescence des complexes.

Certes, des bétaïnes, en très grand nombre, avaient déjà été utilisées pour fonctionnaliser des nano-objets comme des nanoparticules paramagnétiques ou des quantum dots (H. Wei, N. Insin, J. Lee, H.-S. Han, J. M. Cordero, Wenhao Liu, M. G. Bawendi Nano Lett. 2012, 12, 22-25 ; E. Muro, T. Pons, N. Lequeux, A. Fragola, N. Sanson, Z. Lenkei, B. Dubertret J. Am. Chem. Soc 2010, 132, 4556-4557). Cependant, dans ce type de nano-objets polyfonctionnels, il est impossible de savoir si la limitation des phénomènes d'adhésion non spécifiques observés pouvait découler de la nature même du groupe bétaïne ou de la présence simultanée d'un très grand nombre de ces fonctions (phénomène dit de multivalence). Ainsi, en l'état actuel des connaissances, il était totalement impossible de prédire le comportement de petites molécules, tels que des complexes de lanthanides, fonctionnalisées par des groupes bétaïne. Dans la littérature, des groupes bétaïne ont également été introduits sur des chromophores organiques (cyanines, bodipy) pour les rendre solubles dans l'eau, mais rien n'est décrit concernant une éventuelle propriété de non adhésion. La propriété de non adhésion obtenue pour des composés moléculaires, comme c'est le cas dans le cadre de l'invention, n'était donc nullement prévisible.

Un groupe bétaïne est défini comme un groupement zwittérionique dans lequel l'atome portant la charge positive ne porte pas d'atome d'hydrogène et n'est pas adjacent à l'atome porteur de la charge négative. Dans le cadre de l'invention par bétaïne, on entend, par exemple, les groupements zwittérioniques associant un cation ammonium, ou iminium aromatique, généralement pyridinium, imidazollium, et un groupement anionique de type sulfonate, phosphonate ou carboxylate, de préférence sulfonate. Le cation et l'anion sont espacés par au moins un chainon CH₂, et de préférence par une chaine alkyle bivalente (également nommé alkylène) comprenant de 1 à 4, voire de 1 à 6, atomes de carbone. A titre d'exemple de groupe bétaïne, on peut citer les groupes de formule : 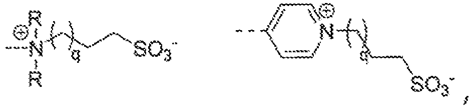 avec R qui représente un groupe alkyle de 1 à 6 atomes de carbone, et de préférence un méthyle ou éthyle, et q qui est égal à 1, 2, 3, 4, 5 ou 6, et de préférence qui est égal à 1 ou 2. Le groupe -N(CH₃)₂⁺-(CH₂)₃-SO₃⁻ est préféré.

De manière avantageuse, les complexes de lanthanide selon l'invention comporte un agent chélatant substitué par au plus 12 groupes bétaïne, par exemple 2, 3, 4, 5, 6, 7, 8, 9, 10 ou 11 groupes bétaïne.

Par ailleurs, les complexes selon l'invention peuvent également être pourvus d'une fonction réactive, pour permettre leur conjugaison par liaison covalente à une molécule d'intérêt, telle qu'une biomolécule. Par fonction réactive, on entend une fonction permettant un greffage covalent sur une fonction réactive présente sur une biomolécule (amine, alcool, thiol, acide carboxylique, fonctions insaturées...). Les différentes fonctions permettant une telle bio-conjugaison sont bien connues de l'Homme de l'Art et sont décrites, par exemple, dans Bioconjugate Techniques, G.T. Hermanson, Academic Press, 1996, 137-166. De manière préférée, cette fonction réactive sera une fonction amine, un ester activé, un groupement azido. En particulier, les complexes de lanthanide selon l'invention comporteront un agent chélatant substitué par au moins une, et en particulier une seule, fonction réactive permettant son couplage par liaison covalente à une biomolécule, la dite fonction réactive étant, de préférence choisie parmi - COOH, -NH₂, un acrylamide, une amine activée, un ester activé, un aldhéhyde, un halogénure d'alkyle, un anhydride, une aniline, un azide, une aziridine, un acide carboxylique, un diazoalcane, un haloacétamide, une halotriazine, une hydrazine, un imido ester, un isocyanate, un isothiocyanate, un maléimide, un halogénure de sulfonyle, un thiol, une cétone, une amine, un halogénure d'acide, un ester d'hydroxysuccinimidyle, un ester de succinimidyle, un ester d'hydroxysulfosuccinimidyle, un azidonitrophényle, un azidophényle, un 3-(2-pyridyl dithio)-propionamide, un glyoxal, une triazine, un groupe acétylénique, et les groupes de formule : 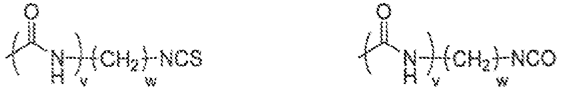 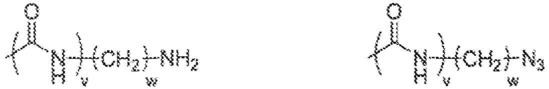 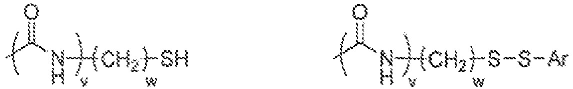 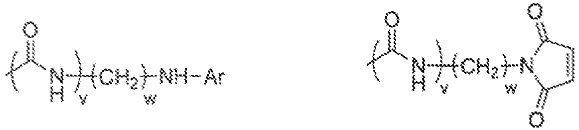 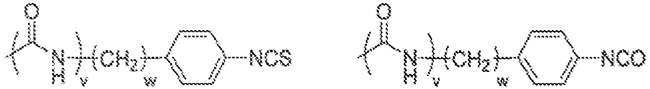 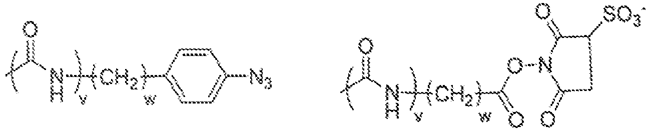 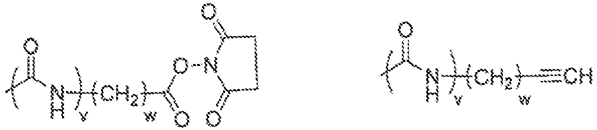 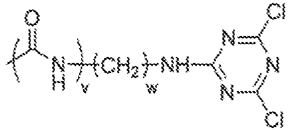
dans lesquels w est un entier appartenant à la gamme allant de 0 à 8 et v est égal à 0 ou 1, et Ar est un hétérocycle à 5 ou 6 chaînons saturé ou insaturé, comprenant 1 à 3 hétéroatomes, éventuellement substitué par un atome d'halogène ;
les fonctions réactives choisies parmi -COOH, -NH₂, les esters de succinimidyle, les haloacétamides, les azides, les hydrazines, les isocyanates et les maléimides étant préférées.

Par biomolécules, on entend les molécules d'intérêt biologique qu'il peut être avantageux de marquer grâce à un complexe luminescent, par exemple, les protéines, les peptides, les anticorps, les antigènes, les brins D'ADN, la biotine, la streptavidine. Les protéines sont les biomolécules qui seront le plus souvent liées aux complexes selon l'invention.

Des exemples de complexes de lanthanide selon l'invention comportent un agent chélatant formé formé d'un macrocycle à structure 1,4,7-triazacyclononane.

En particulier, l'invention concerne les complexes de lanthanide choisis parmi les complexes de lanthanide de formule (IV) : 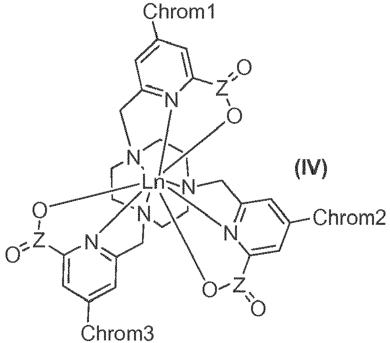 dans lesquelles :
- Ln est un lanthanide, notamment Eu, Sm, Tb ou Dy,
- Z représente -C- ou -PR₃-,
- R₃ représente un groupe phényle, benzyle, méthyle, éthyle, propyle, n-butyle, sec-butyle, iso-butyle ou tert-butyle, et de préférence un groupe phényle ou méthyle,
- Chrom1, Chrom2 et Chrom3, identiques ou différents, sont choisis parmi les groupements : 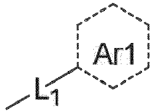 dans lesquels :
   - L₁ représente une liaison directe, -C=C- ou -C≡C-,
   - Ar₁ représente un groupe aromatique choisi parmi les groupes phényle, thiophényle, furanyle, pyrrolyle, imidazolyle ou triazolyle, substitué par s groupes R₀ identiques ou différents,
- s est égal à 1, 2 ou 3, et
- R₀ est choisi parmi les groupes alkyle comprenant de 1 à 10, et de préférence de 1 à 6, atomes de carbone ; les groupes alkyle comprenant de 1 à 10 atomes de carbone porteurs d'au moins une fonction bétaïne et/ou d'une fonction réactive ; et les groupements électrodonneurs, notamment O-donneurs, S-donneurs, NHCO-donneurs, SCO-donneurs, NHCS-donneurs, et SCS-donneurs, lesdits groupes électrodonneurs pouvant être porteurs ou non d'un ou plusieurs groupes bétaïne, et/ou d'une fonction réactive,
étant entendu que lorsque tous les groupes Ar1 représentent un groupe phényle, au moins l'un de ces groupes phényle, de préférence deux, voire trois de ces groupes, est (sont) substitué(s) par au moins un groupe R₀ comportant un groupement électrodonneur ;
caractérisés en ce qu'au moins deux des groupements Chrom1, Chrom2 et Chrom3 sont substitués par au moins un groupe R₀ porteur d'au moins un groupe bétaïne.

De manière préférée, dans les complexes de formule (IV), Chrom1, Chrom2 et Chrom3 sont définis comme suit :
a. Soit Chrom1, Chrom2 et Chrom3, identiques ou différents, sont choisis parmi les groupements : 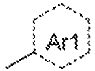 avec Ar1 tel que défini pour les complexes de formule (IV), (ce qui correspond au cas où L₁ représente une liaison directe ou covalente),
b. Soit Chrom1, Chrom2 et Chrom3, identiques ou différents, sont choisis parmi les groupements : 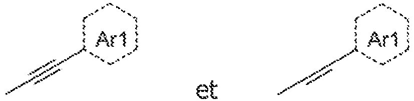 avec Ar1 qui représente un groupe phényle, thiophényle, furanyle, pyrrolyle ou imidazolyle substitué par s groupes R₀ identiques ou différents, s et R₀ étant tels que définis pour les complexes de formule (IV).

Dans les complexes de formule (IV) selon l'invention, les substituants R₀, identiques ou différents, sont choisis, par exemple, parmi :
- -L₂-Alk, -L₂-L₃-Q₁ et -L₂-L₃-Q₂;
- les groupements NHCO-donneurs choisis parmi : -NHCO(OAlk), -NHCO(NHAlk), -NHCO(NAlk1Alk2), -NHCO(SAlk),
- les groupements SCO-donneurs choisis parmi : -SCO(OAlk), -SCO(NHAlk), -SCO(NAlk1Alk2), -SCO(SAlk),
- les groupements NHCS-donneurs choisis parmi : -NHCS(OAlk), -NHCS(NHAlk), -NHCS(NAlk1Alk2), et
- les groupements SCS-donneurs choisis parmi : -SCS(OAlk), -SCS(NHAlk), -SCS(NAlk1Alk2), -SCS(SAlk),
- Alk, Alk1 et Alk2, identiques ou différents, sont des groupes alkyle comprenant de 1 à 10 atomes de carbone, éventuellement substitués par au moins un groupe bétaïne,
- Q₁ représente un groupe bétaïne ou un groupement ramifié porteur d'au moins deux groupes bétaïne,
- L₂ est une liaison directe, -O-, -S-, -NHCO-, -SCO-, -NHCS- ou -SCS-
- L₃ est un bras de liaison, et
- Q₂ est un groupe réactif susceptible de permettre la liaison covalente avec une molécule d'intérêt à marquer,
étant entendu qu'au moins deux des substituants R₀ présents sont porteurs d'au moins un groupe bétaïne, de manière à ce qu'au moins deux des groupements Chrom1, Chrom2 et Chrom3 soient substitués par au moins un groupe R₀ porteur d'au moins un groupe bétaïne.

A titre d'exemple de groupement ramifié porteur d'au moins deux groupes bétaïne, on peut citer les groupes de formule suivante : 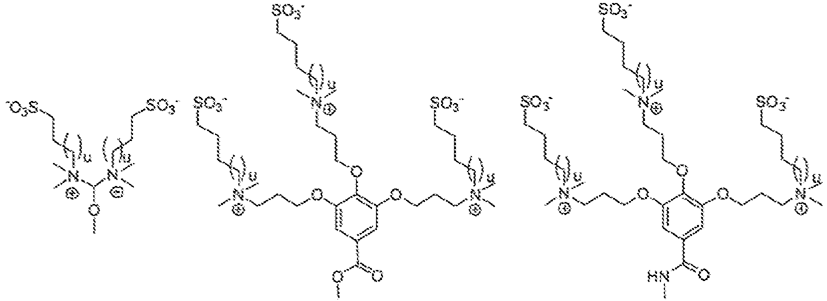 avec u qui est égal à 1, 2, 3, 4, 5 ou 6.

Dans les complexes de formule (iv) selon l'invention, il est possible que Chrom1 = Chrom2 = Chrom3 ou, de préférence, que Chrom1 = Chrom2 et soient substitués par au moins un groupe R₀ porteur d'au moins un groupe bétaïne et que Chrom3 soit substitué par au moins un groupement R₀ porteur d'une fonction -L₂-L₃-Q₂, tel que défini précédemment, avec de préférence Q₂ qui représente un groupe choisi parmi -COOH, -NH₂, un acrylamide, une amine activée, un ester activé, un aldhéhyde, un halogénure d'alkyle, un anhydride, une aniline, un azide, une aziridine, un acide carboxylique, un diazoalcane, un haloacétamide, une halotriazine, une hydrazine, un imido ester, un isocyanate, un isothiocyanate, un maléimide, un halogénure de sulfonyle, un thiol, une cétone, une amine, un halogénure d'acide, un ester d'hydroxysuccinimidyle, un ester de succinimidyle, un ester d'hydroxysulfosuccinimidyle, un azidonitrophéniyle, un azidophényle, un 3-(2-pyridyl dithio)-propionamide, un glyoxal, une triazine, un groupe acétylénique, et les groupes de formule : 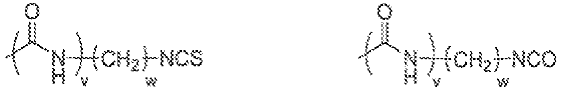 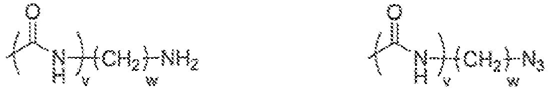 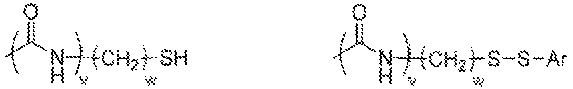 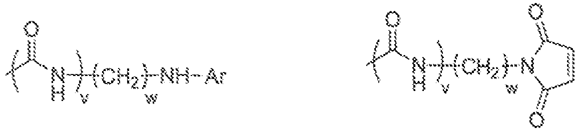 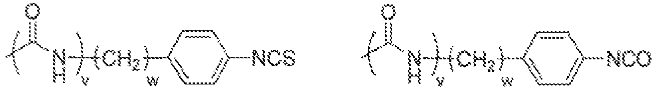 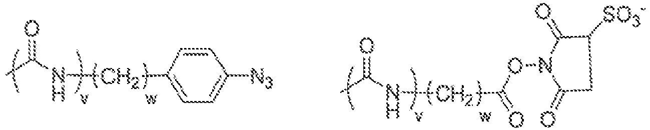 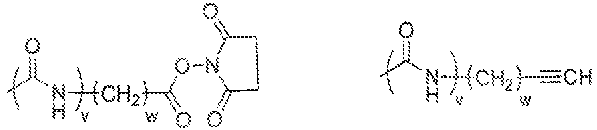 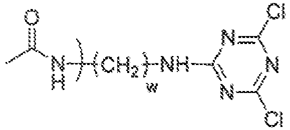
dans lesquels w est un entier appartenant à la gamme allant de 0 à 8 et v est égal à 0 ou 1, et Ar est un hétérocycle à 5 ou 6 chaînons saturé ou insaturé, comprenant 1 à 3 hétéroatomes, éventuellement substitué par un atome d'halogène ;
Q₂ étant de préférence choisi parmi -COOH, -NH₂, les esters de succinimidyle, les haloacétamides, les hydrazines, les isocyanates et les maléimides étant préférées.

Les complexes de formule (IV) selon l'invention, dans lesquels L₁ représente une liaison directe ou -C≡C-,et les groupes Ar1, identiques ou différents, représentent chacun un groupe phényle, substitué par s groupes R₀ identiques ou différents, avec s et R₀ qui sont tels que définis pour les complexes de formule (IV), sont des exemples de complexes selon l'invention.

Les complexes de formule (IV) selon l'invention dans lesquels s est égal à 1 pour tous les groupes Ar1 sont plus simples à synthétiser et seront préférés.

A titre d'exemple de complexes de formule (IV) selon l'invention, on peut citer ceux dont tous les groupes Ar1, identiques ou différents, représentent un groupe phényle choisi parmi les groupements : 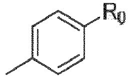 avec R₀ tel que défini pour les complexes de formule (IV).

Dans les complexes de formule (IV) selon l'invention, L₃ représente, de préférence, une liaison covalente, un groupe alkylène de 1 à 12 atomes de carbone, comprenant éventuellement une ou plusieurs double ou triple liaison; un groupe cycloalkylène de 5 à 8 atomes de carbone, un groupe arylène de 6 à 14 atomes de carbone ; ou un enchainement de un ou plusieurs groupes alkylène de 1 à 12 atomes de carbone, cycloalkylène de 5 à 8 atomes de carbone et/ou arylène de 6 à 14 atomes de carbone ; lesdits groupes alkylène, cycloalkylène ou arylène pouvant ou non comprendre un ou plusieurs hétéroatomes tels que l'oxygène, l'azote, le soufre, le phosphore ou un ou plusieurs carbamoyle ou carboxamido et/ou pouvant être non substitués ou substitués par un ou plusieurs groupe alkyle de 1 à 8 atomes de carbone, aryle de 6 à 14 atome de carbone, sulfonate ou oxo ; les bras de liaison suivants étant préférés : 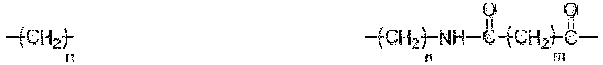 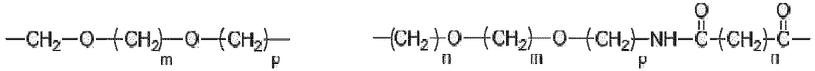 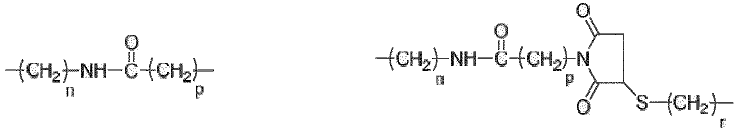 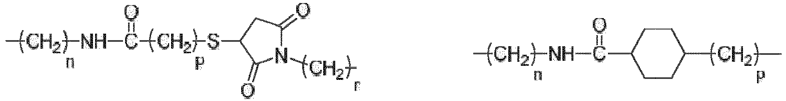 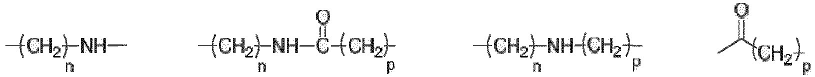 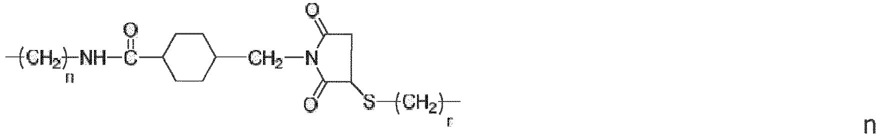 est égal à 1, 2, 3, 4, 5 ou 6,
m, p et r, identiques ou différents, sont égaux à 1, 2 ou 3.

Lorsque L₂ et L₃ représentent chacun une liaison directe (nommée indifféremment liaison covalente), -L₂-L₃-Q₁, correspond à -Q₁ et -L₂-L₃-Q₂ à -Q₂

Dans les complexes de formule (IV) définis dans le cadre de l'invention, si Ln³⁺= Eu³⁺ ou Sm³⁺, dans ce cas, on choisira, selon un mode de réalisation préféré, L₁ qui représente -C≡C-,qui conduit à des propriétés de luminescence appropriées.

Dans les complexes de formule (IV) définis dans le cadre de l'invention, si Ln³⁺₌ Eu³⁺, Sm³⁺, Tb³⁺ ou Dy³⁺ (et, en particulier Tb³⁺ ou Dy³⁺), on choisira, selon un mode de réalisation préféré, L₁ qui représente une liaison directe (nommée également liaison covalente).

Dans les complexes de formule (IV) définis dans le cadre de l'invention, les groupes bétaïne présents sont, par exemple, des groupes zwittérioniques associant un cation ammonium, ou iminium aromatique, généralement pyridinium, imidazollium, et un groupement anionique de type sulfonate, phosphonate ou carboxylate, de préférence sulfonate. Le cation et l'anion sont espacés par au moins un chainon CH₂, et de préférence par une chaine alkyle bivalente (également nommé alkylène) comprenant de 1 à 4, voire de 1 à 6, atomes de carbone. De préférence, les groupes bétaïne présents dans les complexes de formule (IV) et (III) sont choisis parmi : 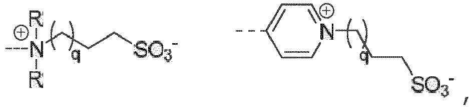 avec R qui représente un groupe alkyle de 1 à 6 atomes de carbone, et de préférence un méthyle ou éthyle, et q qui est égal à 1, 2, 3, 4, 5 ou 6, et de préférence qui est égal à 1 ou 2, le groupe -N(CH₃)₂⁺-(CH₂)₃-SO₃⁻ étant préféré. L'invention a également pour objet :
- les agents chélatants de formule (II) : 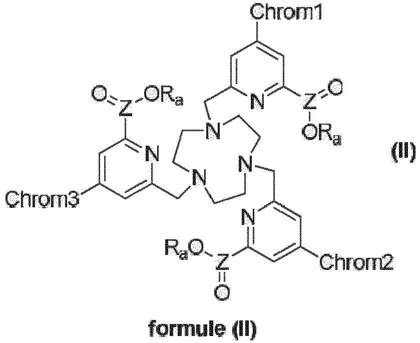 avec Ra qui est un groupe protecteur des fonctions acides tel qu'un alkyle, du type méthyle, et Z, Chrom1, Chrom2 et Chrom3, identiques ou différents, qui sont tels que définis pour les composés de formule (IV)

Dans le cadre de l'invention, par groupe « alkyle », à moins qu'il n'en soit spécifié autrement, on entend une chaîne hydrocarbonée, saturée, linéaire ou ramifiée. A titre d'exemple de groupe alkyle comprenant de 1 à 6 atomes de carbone, on peut citer, notamment, les groupes méthyle, éthyle, *n*-propyle, *iso-*propyle, *n*-butyle, *tert*-butyle, *sec*-butyle, n-pentyle, n-hexyle.

Par « cycloalkyle », on entend un groupement hydrocarboné saturé ou insaturé constitué d'au moins un cycle, éventuellement ponté. A titre d'exemple, on peut citer les groupes cyclopropyle, cyclobutyle, cyclopentyle, cyclohexyle, cycloheptyle, adamantyle et cyclohexene.

Par groupe « aryle », on entend les groupes carbocycliques mono-, bi- ou polycycliques, comportant de préférence de 6 à 12 atomes de carbone, comprenant au moins un groupe aromatique, par exemple, un groupe phényle, cinnamyle ou naphtyle ; ainsi que les groupes hétéroaryle. Par groupe hétéroaryle, on entend un carbocycle mono-, bi- ou polycyclique, comportant de préférence de 6 à 12 chainons, et comprenant au moins un groupe aromatique et au moins un hétéroatome choisi parmi les atomes d'oxygène, azote ou soufre intégré au sein du carbocycle. A titre d'exemple de groupe hétéroaryle, on peut citer les 2-, 3- ou 4-pyridinyle, 2- ou 3-furoyle, 2- ou 3-thiophényle, pyrrolyle, imidazolyle, pyrazolyle, thiazolyle, oxazolyle, isoxazolyle, pyridinyle, pyrazinyle, pyrimidinyle, tétrazolyle, thiadiazolyle, oxadiazolyle, triazolyle, pyridazinyle, indolyle. Le phényle et le 1,2,3-triazole sont les groupes aryle particulièrement préférés.

De manière avantageuse, les complexes selon l'invention sont luminescents. La luminescence correspond à une émission de lumière consécutive à un apport en énergie, en particulier par un apport de lumière. Cet apport en énergie fait passer des atomes ou molécules dans un état « excité » se situant à une énergie supérieure à celle qu'ils possèdent dans leur état normal dit « fondamental ». C'est lors du retour à leur état fondamental qu'ils peuvent émettre de la lumière. De manière générale, les
complexes de lanthanide sont sensibilisés par effet d'antenne et la nature de l'antenne organique contrôle l'absorption de la lumière et permet d'optimiser la luminescence du lanthanide (S. V. Eliseeva, J.-C. G. Bünzli, Chem. Soc. Rev. 2010, 39, 189).

Dans le cadre de l'invention, l'antenne est, de manière avantageuse, constituée d'un dérivé de pyridine fonctionnalisé en position 4 par un système conjugué appelé chromophore et défini notamment sous les dénominations Chrom1, Chrom2, Chrom3 et Chrom4 dans les complexes préférés. Ce chromophore est constitué éventuellement d'un espaceur conjugué choisi parmi les double ou triple liaisons carbone-carbone, qui de manière avantageuse correspond à la liaison -C≡C-, et d'un groupe aryle ou heteroaryle substitué par 1, 2 ou 3 groupements, de manière à ce qu'au moins 2 groupes bétaïne soient présents sur le complexe final. Les antennes dans lesquelles le groupe aryle ou heteroaryle est directement lié à la pyridine seront préférées pour sensibiliser des complexes d'europium, samarium, terbium et dysprosium, alors que les antennes comportant un espaceur conjugué seront préférées pour les complexes d'europium et de samarium.

Plus particulièrement, l'invention est appliquée à des macrocycles chélatants décrits sous leur forme protégée selon la formule (II) et à leurs complexes de lanthanide correspondant selon la formule (IV).

Selon la formule (IV) est décrite la famille de complexes de lanthanide issue de la coordination d'un ligand macrocyclique de formule (II) après déprotection et libération des fonctions acide.

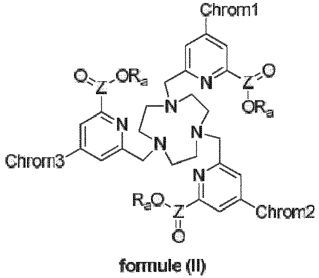

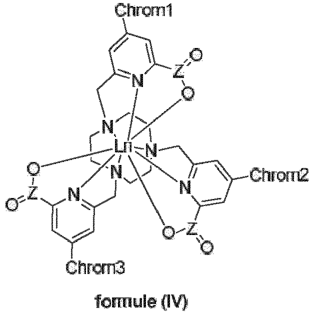

A titre d'exemple de sous-structure des complexes de formule (IV) tel qu'exemplifiés dans les exemples, on peut citer :
a) Les complexes (IVa) 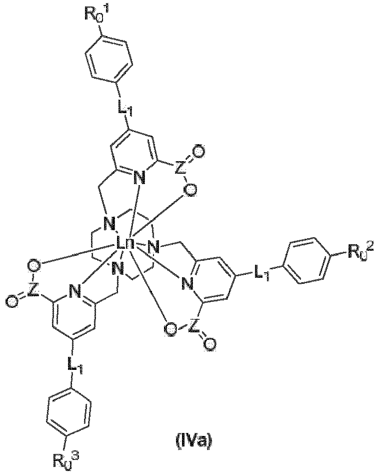
   avec L₁ tel que défini dans le cadre de l'invention et qui représente de préférence -C≡C-,Z tel que défini pour les complexes de formule (IV) et qui représente de préférence un atome de carbone et R₀¹,
   R₀² et R₀³, identiques ou différents, qui représentent un groupe R₀ tel que défini pour les complexes de formule (IV), au moins deux des groupes R₀¹, R₀² et R₀³ étant substitués par au moins un groupe bétaïne,

La préparation des complexes selon l'invention met en oeuvre des techniques et réactions classiques connues de l'homme de l'art. Ils peuvent, notamment être obtenus selon des procédés analogues à ceux utilisés dans les exemples.

Les réactifs de départ sont disponibles dans le commerce ou facilement accessibles. Les composés de formule (II) sont donc d'accès chimique relativement classique, et peuvent être obtenus à un coût de préparation raisonnable.

Les groupes fonctionnels éventuellement présents dans les composés de formule (II) et dans les intermédiaires réactionnels peuvent être protégés au cours des synthèses, soit sous forme permanente soit sous forme temporaire, par des groupes protecteurs qui assurent une synthèse univoque des composés attendus. Les réactions de protection et déprotection sont effectuées selon des techniques bien connues de l'Homme de l'art. Par groupe protecteur temporaire des amines, alcools ou des acides carboxyliques, on entend les groupes protecteurs tels que ceux décrits dans Protective Groups in Organic Synthesis, Greene T.W. et Wuts P.G.M., ed John Wiley et Sons, 2006 et dans Protecting Groups, Kocienski P.J., 1994, Georg Thieme Verlag.

Les réactions de complexation sont, en général, menées avec un sel du lanthanide souhaité notamment un chlorure, un nitrate ou un triflate dans un solvant dissociant, notamment un alcool et, de manière préférée, le méthanol ou l'acétonitrile, ou encore dans un mélange de solvants, en présence d'une base généralement un carbonate, à une température comprise entre 25 et 80°C, pendant une durée variant de 30 minutes à quelques heures.

Par exemple, dans le cas des complexes (IV), les agents complexants de formule (II) selon l'invention, dans le cas où les trois chromophores sont identiques (agents complexants symétriques) peuvent être préparés à partir de l'intermédiaire (A1) : 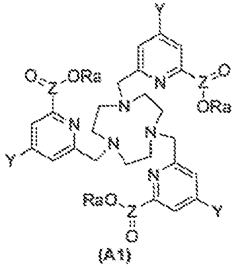 dans laquelle Ra est un groupe protecteur des fonctions acide tel qu'un alkyle de 1 à 4 atomes de carbone, du type méthyle, Z est tel que défini pour les composés de formule (IV) et Y qui représente un atome de chlore, brome, ou de préférence iode, ou une fonction -N₃ ou -C≡CH.

Les agents complexants de formule (II) selon l'invention, dans le cas où Chrom1 = Chrom2 ≠ Chrom3 (agents complexants dissymétriques) peuvent être préparés à partir de l'intermédiaire (A2) : 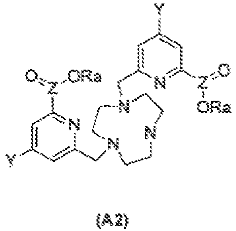 dans laquelle Ra est un groupe protecteur des fonctions acide tel qu'un alkyle de 1 à 4 atomes de carbone, du type méthyle, Z est tel que défini pour les composés de formule (IV) et Y qui représente un atome de chlore, brome, ou de préférence iode, ou un une fonction -N₃ ou -C≡CH.

L'introduction des groupements Chrom1, Chrom2 et Chrom3 peut se faire en une seule étape dans le cas des agents complexants symétriques à partir de (A1) ou en deux étapes dans le cas des agents complexants dissymétriques à partir de (A2) selon les techniques détaillées dans les exemples ci-après, ou selon des techniques analogues bien connues de l'homme de l'art.

Dans le cadre de l'invention, le procédé mettant en oeuvre une synthèse divergente à partir des intermédiaires (A1) et (A2) clefs est plus rapide et plus efficace pour la préparation d'agents complexant de formule (IV) que le procédé proposé pour la préparation des agents complexants dans la demande WO 2013/011236. Dans le procédé de synthèse utilisé dans le cadre de l'invention, les chromophores Chrom1, Chrom2 et Chrom3 ou certains de leurs précurseurs sont introduits directement sur (A1) et (A2) en utilisant des réactions classiques de couplage carbone-carbone catalysées au palladium, comme la réaction de Sonogashira, de Heck, de Susuki, de Stille, ou encore les réactions de cycloaddition 1,3 dipolaire catalysée au cuivre appelées communément « chimie click ».

Dans tous les cas, les chromophores Chrom1, Chrom2 et Chrom3 seront fonctionnalisés par des fonctions réactives compatibles avec les réactions de couplage carbone-carbone ou de « chimie click » envisagées. Ces fonctionnalisations sont conduites selon des protocoles issus de la littérature et connus de l'homme de l'art. Est envisagée notamment la présence de fonctions alcyne ou alcène terminale dans le cas des chromophores Chrom1, Chrom2 et Chrom3 présentant un espaceur, compatibles avec des réactions de Sonogashira et de Heck. Est envisagée également la présence d'une fonction acide boronique compatible avec des réactions de Suzuki dans le cas des chromophores Chrom1, Chrom2 et Chrom3 sans espaceur dans lesquelles le groupe aryle ou hétéroaryle est directement lié à la pyridine.

Notamment, l'introduction de groupements : 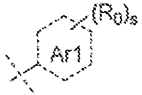 avec Ar1, s et R₀ (étant entendu que différents groupes R₀ peuvent substituer le groupe Ar1) qui sont tels que définis pour les composés de formule (IV), pourra se faire :
- soit par action d'un composé de formule (V): 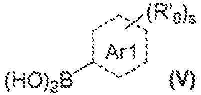 avec R'₀ (étant entendu que différents groupes R'₀ peuvent substituer le groupe Ar1) qui représente R₀ ou un groupe précurseur de R₀ et Ar1, s et R₀ qui sont tels que définis pour les composés de formule (IV), sur l'intermédiaire (A1) ou (A2), dans lequel Y est un atome de chlore, brome ou de préférence iode, dans les conditions d'un couplage de Sukuzi,
- soit dans le cas où Ar1 représente un groupe 1,2,3-triazole, par action d'un composé de formule (VI) : 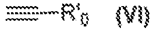 avec R'₀ qui représente R₀ ou un groupe précurseur de R₀, R₀ étant tel que défini pour les composés de formule (IV), sur l'intermédiaire (A1) ou (A2), dans lequel Y est N₃, par une réaction dite de «chimie click ». Il est également possible de réaliser le couplage avec un composé N₃-R'₀, la fonction alcyne étant alors portée par la pyridine de l'intermédiaire (A1) ou (A2).

L'introduction de groupements : 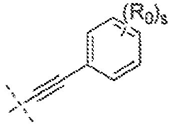 avec R'₀ (étant entendu que différents groupes R'₀ peuvent substituer le groupe phényle) qui représente R₀ ou un groupe précurseur de R₀, et R₀ et s qui sont tels que définis pour les composés de formule (IV), sur l'intermédiaire (A1) ou (A2) dans lequel Y est un atome de chlore, brome ou de préférence iode, dans les conditions d'une réaction de Sonogashira.

Quelle que soit la voie de synthèse envisagée et le complexe de lanthanide visé, les groupes bétaïne peuvent, par exemple, être introduites au niveau des substituants R₀ ou R₁ *via* une alkylation directe d'un groupement phénol, thiophénol, aniline etc... ou bien *via* une réaction dite de « chimie click» à partir d'un précurseur propargylique.

Pour former les complexes de lanthanide correspondants, le macrocycle de formule (II) est mis en solution dans un solvant dans lequel il est soluble, par exemple de l'éthanol ou du méthanol, et la déprotection des fonctions acide est réalisée en milieu basique, puis la complexation est menée avec un sel de la terre rare souhaitée, notamment sous la forme d'un chlorure, triflate ou nitrate. De façon avantageuse, environ 1 équivalent molaire de macrocycle de formule (II) est utilisé par atome de terre rare. La complexation peut être réalisée à une température de 25 à 80 °C, par exemple pendant une durée de l'ordre de 30 minutes à 6 heures.

Les exemples ci-après permettent d'illustrer l'invention, mais n'ont aucun caractère limitatif.

Les abréviations ci-dessous sont utilisées dans les exemples qui vont suivre :
ACN : Acétonitrile
DMF : Diméthylformamide
DPA : Acide dipicolinique
Et₃N : Triéthyle amine
MeOH : Méthanol
Ms : Groupement Mésyle
TA : Température Ambiante
TACN : Triazacyclononane
TBTA : tris[(1-benzyl-1*H*-1,2,3-triazol-4-yl)méthyl]amine
THF : Tétrahydrofurane
TMSA : Triméthylsilylacétylène

### Informations Générales :

Les spectres RMN (¹H et ¹³C) ont été enregistrés sur un appareil Bruker AC 200 à une fréquence de 200.13 et 50.32 MHz pour les ¹H et ¹³C respectivement et sur un Bruker Advence à une fréquence de 500.10 et 125.75 MHz pour des ¹H et ¹³C respectivement Les déplacements chimiques (δ) sont exprimés en partie par million (ppm) par rapport au triméthylsilane utilisé comme référence interne et en utilisant les solvants indiqués. Les constantes de couplage (*J*) sont exprimés en Hz et les notations suivantes sont utilisées: s (singulet), brs (broad singulet ou singulet large), d (doublet), dd (doublet de doublet), m (multiplet).

Les spectres de masse haute résolution HRMS ont été enregistrés au centre commun de spectrométrie de masse de Lyon (Université Claude Bernard Lyon, Lyon, France) sur un appareil MicroTOFQII équipé avec une source ESI positive.

Les chromatographies sur couche mince ont été réalisées sur des plaques de gel de silice sur feuilles d'aluminium (silica gel, Fluka) et révélées à l'aide d'une lampe UV (λ = 254 ou 365 nm) ou bien par coloration.

Les purifications ont été réalisées par colonne chromatographique sur gel de silice (silica gel 0.035-0.070 mm, 60A).

Les solvants utilisés pour les réactions ont été achetés chez Aldrich ou Acros Organics en tant que solvants sec ou extra sec et stockés sur tamis moléculaire 3Å.

Les spectres d'absorption UV/Vis ont été enregistrés sur un spectromètre JASCO V670 ; les spectres d'émission sur un spectrofluorimètre JOBIN-YVON fluorolog 3.

### A. PREPARATION DES COMPLEXES

### Exemple 1 : Complexe d'Europium de formule (IV)

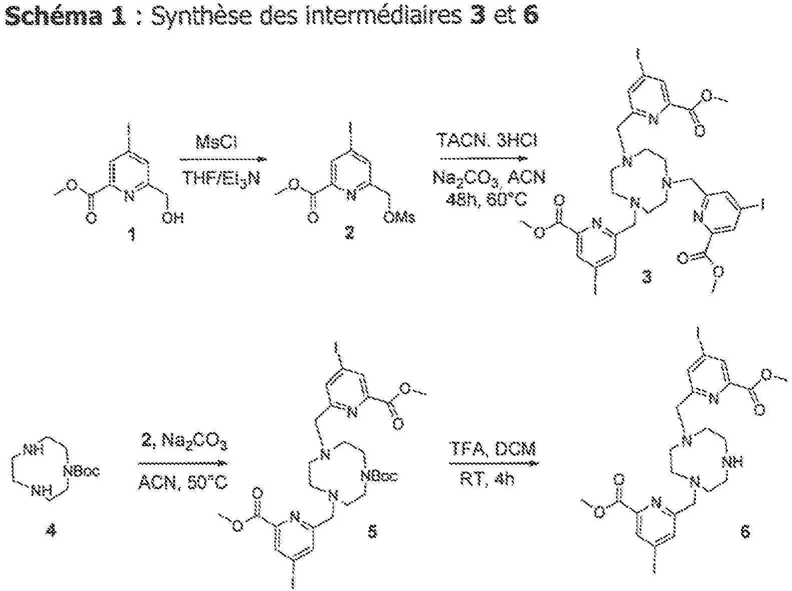

L'intermédiaire **3** est obtenu par une réaction d'alkylation du TACN.3HCl, à partir d'un dérivé mésylate (ou tosylate) dans les conditions classiques d'alkylation. Ce composé va pouvoir ensuite être engagé dans diverses réactions de couplage croisé pour obtenir les ligands désirés.

La synthèse de l'intermédiaire dissymétrique **6** nécessite plusieurs étapes de protection/déprotection du TACN et est préparé conformément à la méthode décrite dans le brevet WO 2013/011236.

Le TACN-boc **4** est substitué par le composé **2** pour mener au composé **5** précurseur de l'intermédiaire dissymétrique **6** après déprotection de la fonction Boc.

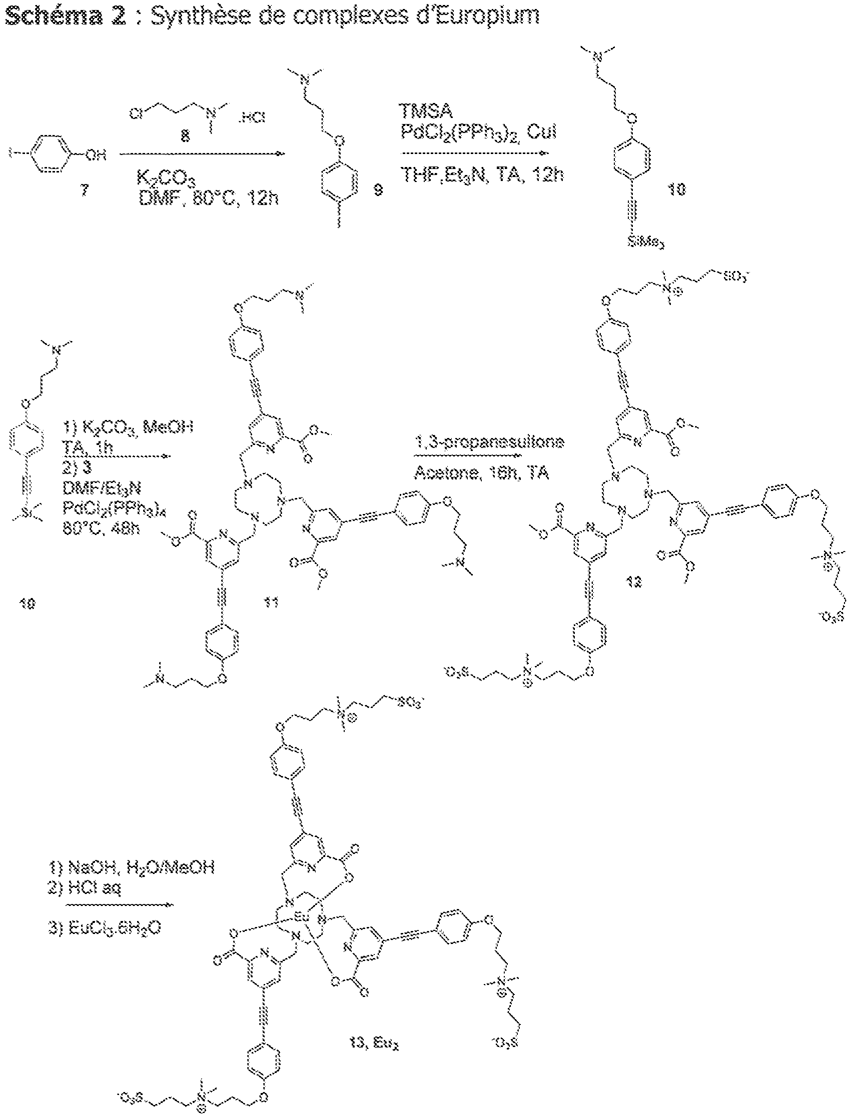

La synthèse du composé **10** peut être réalisée dans des conditions classiques de substitution sur le 4-iodophénol à partir d'une amine commerciale **8.** La fonction alcyne protégée du composé **10** peut être introduite *via* une réaction de couplage croisé de Sonogashira en présence de TMSA. L'introduction du composé **10** sur l'intermédiaire **3** peut être réalisée après déprotection de l'alcyne, en alcyne vrai, qui est ensuite engagé directement dans une étape de couplage de Sonogashira modifié (sans CuI). La fonction bétaïne peut être préparée par une réaction d'ouverture de la 1,3-propanesultone sélectivement sur l'amine terminale du composé **11** pour mener à l'agent complexant **12.** Les esters méthyliques peuvent ensuite être hydrolysés et l'agent complexant obtenu réagit *in situ* en présence d'une solution de sel d'europium pour mener au complexe **Eu2.** 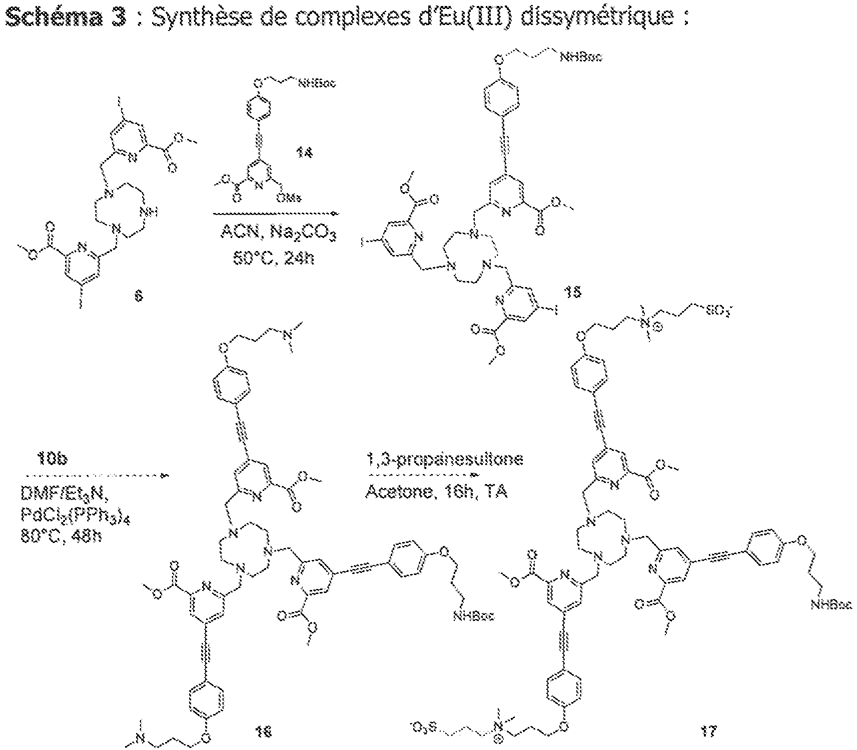 Le composé **15** est obtenu après substitution du composé **6** par l'antenne **14.** Le composé **14** est obtenu comme décrit dans la demande de brevet WO 2013/011236. Le composé **16** pourra être obtenu après couplage de Sonogashira de l'antenne **10b**, tel que décrit précédemment pour le complexe symétrique. De même le composé **17** pourra être obtenu après ouverture de la 1,3-propanesultone sélectivement sur les amines terminales du composé **16.**

### a) Préparation du composé 2

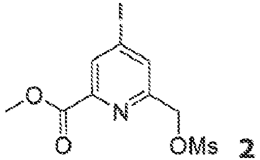

Le composé 1 (1,56g, 5,33mmol, 1,0eq) est dissous dans 50 mL de THF distillé. La triéthylamine (1,63 mg, 16 mmol, 3,0eq) est ensuite ajoutée, ainsi que le chlorure de mésyle (976 mg, 8,53 mmol, 1.6 eq). La solution est agitée sous argon à température ambiante. La réaction est suivie par CCM. Après 4h, la réaction est totale, les solvants sont évaporés. Le résidu est solubilisé dans l'acétate d'éthyle et la phase organique est lavée avec une solution saturée de NaHCO₃, puis à l'eau. La phase organique est ensuite séchée sur Na₂SO₄, puis filtrée. Le solvant est éliminé sous pression réduite. Le produit brut obtenu est purifié par colonne chromatographie sur gel de silice (Eluant : dichlorométhane/méthanol de 0% jusqu'à 5% en volume, par incrément de 1%) conduisant au produit désiré sous la forme d'un solide blanc (1,40 g, 90%).
**RMN ¹H (500 MHz, CDCl₃) δ**: 8,47 (d, ⁴*J*=1,5 Hz, 1H), 8,05 (d, ⁴*J*=1,5 Hz, 1H), 5,37 (s, 2H), 4,00 (s, 3H), 3,16 (s, 3H)
**RMN ¹³C (500 MHz, CDCl₃) δ**: 164, 155, 148, 134, 107, 70, 53, 38 **HRMS (ESI** ⁺) : (Calc pour C₉H₁₀NO₅SNa : 393,9212) ; mesurée : [M+Na]⁺ : m/z= 393,9217.

### b) Préparation du composé 3

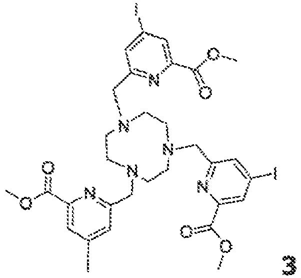

A une suspension de Na₂CO₃ (1,59g, 15mmol, 10eq) dans 60 ml d'acétonitrile sous argon sont ajoutés le TACN.3HCl (356mg, 1,5mmol, 1,0eq), et le composé **2** (1,4g, 4,79mmol, 3,2eq). Le mélange réactionnel est agité 48h à 60°C sous argon. Après retour à température ambiante, le mélange réactionnel est filtré et le filtrat est évaporé sous pression réduite. Le brut réactionnel est purifié par chromatographie sur colonne d'alumine neutre (Eluant : acétate d'éthyle). Le produit final est obtenu sous la forme d'un solide jaune (814 mg, 57%).
**RMN ¹H** (200 **MHz, CDCl₃) δ**: 8,35 (d, *J*=1,4 Hz, 3H), 8,21 (d, *J*=1,4 Hz, 3H), 3,97 (s, 9H), 3,93(s, 6H), 2,90 (s, 12H)
**RMN ¹³C (125,76 MHz, CDCl₃) δ:** 164,6, 161,9, 147,65, 147,62, 135,6, 132,8, 106,65, 106,60, 63,9, 56,1, 53,2, 53,19, 53,14.
**HRMS (ESI ⁺)** : (Calc pour C₃₀H₃₄I₆O₆ : 954,9627); mesurée : [MH]⁺: m/z= 954,9668.

### c) Préparation du composé 5

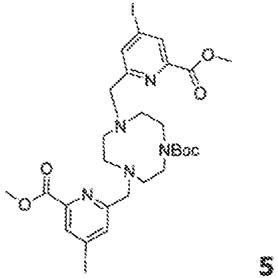

Dans un ballon, le composé **4** (370mg, 1,24mmol, 1.0 eq) est solubilisé dans de l'acétonibile sec (100mL). A cette solution sont ajoutés sous argon le Na₂CO₃ (789mg, 7,44 mmol, 6,0eq) et le composé 3 (966 mg, 2,6 mmol, 2,1 eq). Le mélange réactionnel est agité sous argon à 60°C pendant 4h. A la fin de la réaction, le Na₂CO₃ est filtré sous pression réduite et les solvants sont évaporés. Le brut réactionnel est purifié sur colonne chromatographique sur gel de silice (Eluant : AcOEt). Le produit est obtenu sous la forme d'une huile jaune (633 mg, 66%).
**RMN ¹H (500 MHz, CDCl₃) δ**: 8,35 (2s, 2H), 8,24 (s, 1H), 8,13 (s, 1H), 3,98 (2s, 2x 3H), 3,95 (2s, 2x 1H), 3,42 (brs, 2H), 3,36 (brs, 2H), 3,10 (brs, 2H), 2,98 (brs, 2H), 2,7 (brs, 2H), 2,62 (brs, 2H), 1,48 (s, 9H).
**RMN ¹³C (125,76 MHz, CDCl₃) δ:** 164,80, 164,73, 162,32, 162,13, 155,79, 147,95, 147,77, 135,57, 135,39, 132,96, 132,92, 106,81, 106,76, 79,69, 63,30, 63,03, 56,77, 55,15, 55,10, 54,59, 53,28, 50,94, 50,42, 28,91.

### d) Préparation du compose 6

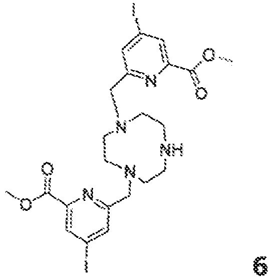

Dans un ballon, le compose **5** (50mg, 6,41.10-2mmol, 1,0 eq) est solubilisé dans du CH₂Cl₂ (2mL). A cette solution est ajouté l'acide trifluoroacétique (20eq). Le mélange réactionnel est agité sous argon pendant 4h. A la fin de la réaction, les solvants sont évaporés sous pression réduite. Le produit est obtenu sous la forme d'une huile jaune et est engagé directement dans retape suivante sans autre purification.
**RMN ¹H (500 MHz, CDCl₃) δ:** 8,29(s, 2H), 7,88 (s, 2H), 4,34 (brs, 4H), 3,89 (s, 6H), 3,65 (brs, 4H), 3,51 (brs, 4H), 3,29 (brs, 4H).

### e) Préparation du composé 9

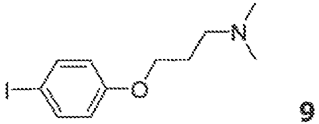

Dans un ballon, le composé **7** (1,00g, 4,54mmol, 1,0eq) est solubilisé dans du DMF anhydre (30mL). A cette solution sont ajoutés l'amine 8 (1,07g, 6,81mmol, 1,5eq) et K₂CO₃ (6,30 g, 45,4 mmol, 10 eq). Le mélange réactionnel est agité sous argon à 80°C pendant 12h. Après retour à la température ambiante, du CH₂Cl₂ est ajouté. La phase organique est lavée trois fois avec une solution aqueuse de NaCl saturée, puis à l'eau, séchée sur Na₂SO₄, filtrée et évaporée. Le produit est obtenu sous la forme d'une poudre jaune sans autre purification (1,20g, 87%).
**RMN ¹H (200 MHz, CDCl₃) δ:** 7.54 (d, 2H, *J*= 8.9Hz), 6.73 (d, 2H, *J*=8.9Hz), 3.97(t, 2H, *J*= 6.4Hz), 2.43 (t, 2H, *J*= 6.9Hz), 2.25 (s, 6H), 1.93 (m, 2H).
RMN **¹³C** (50.32 **MHz, CDCl₃) δ:** 159.04, 138.26, 117.07, 82.65, 66.47, 56.43, 45.67, 27.60.
**HRMS (ESI ⁺) :** (Calc pour C₁₁H₁₆INO : 305.0277) ; mesurée : [MH]⁺ : m/z= 306.0349

### f) Préparation du composé 10

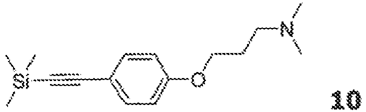

Dans un tube de Schlenk, le composé **9** (860mg, 2,81mmol, 1,0eq) est solubilisé dans un mélange THF/Et₃N (20mL, 1/1, v/v). La solution est dégazée sous argon pendant 20min puis le TMSA (555mg, 5,64mmol, 2,0eq) est ajouté, ainsi que les catalyseurs, PdCl₂(PPh₃)₂ (59mg, 7,86.10⁻⁵mol, 0,03 eq) et CuI (50 mg, 2,64. 10⁻⁴ mol, 0,1eq). Le mélange réactionnel est agité sous argon à la température ambiante pendant 16h. A la fin de la réaction, les solvants sont évaporés sous pression réduite. Le brut réactionnel est dissous dans du CH₂Cl₂ et la phase organique est lavée avec une solution aqueuse saturée en NH₄Cl, puis à l'eau. La phase organique est ensuite séchée sur Na₂SO₄, filtrée et évaporée sous pression réduite. Le brut obtenu est purifié sur colonne chromatographique sur gel de silice. Eluant CH₂Cl₂/MeOH (de 0 à 5% en volume). Le composé désiré est obtenu sous la forme d'une huile orange (650mg, 84%).
**RMN ¹H (200 MHz, CDCl₃) δ:** 7.36 (d, 2H, *J*= 8.9Hz), 6.78 (d, 2H, *J*=8.9Fz), 3.97(t, 2H, *J*= 6.4Hz), 2.43 (t, 2H, *J*= 7.3 Hz), 2.24 (s, 6H), 1.93 (m, 2H), 0.22 (s, 9H).
**RMN ¹³C (50.32 MHz, CDCl₃) δ**: 159.33, 133.54, 115.21, 115.21, 114.46, 105.42, 92.39, 66.32, 56.42, 45.66, 27.61.
**HRMS (ESI** ⁺) : (Calc pour C₁₆H₂₅OSi : 275.1705) ; mesurée : [MH]⁺ : m/z= 276.1805

### g) Préparation du composé 10b

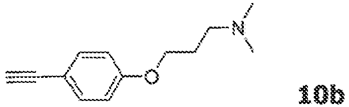

Dans un ballon, le composé **10** (445mg, 1,61mmol, 1,0eq) est solubilisé dans du MeOH anhydre (15 mL). A cette solution est ajouté du K₂CO₃ (268mg, 1,93mmol, 1,2eq). Le mélange réactionnel est agité à la température ambiante pendant une heure. A la fin de la réaction, le solvant est évaporé sous pression réduite. Le résidu est solubilisé dans du CH₂Cl₂ et la phase organique est lavée deux fois à l'eau, puis séchée sur Na₂SO₄, filtrée et évaporée sous pression réduite. Le composé est obtenu sous la forme d'une huile brune et est engagé directement dans l'étape suivante sans autre purification.
**RMN ¹H (200 MHz, CDCl₃) δ**: 7.36 (d, 2H, J= 8.9Hz), 6.84 (d, 2H, J=8.9Hz), 4.02 (t, 2H, J= 6.5Hz), 2.99 (s, 1H), 2.44 (t, 2H, J= 7.0 Hz), 2.25 (s, 6H), 1.95 (m, 2H).

### h) Préparation du composé 11

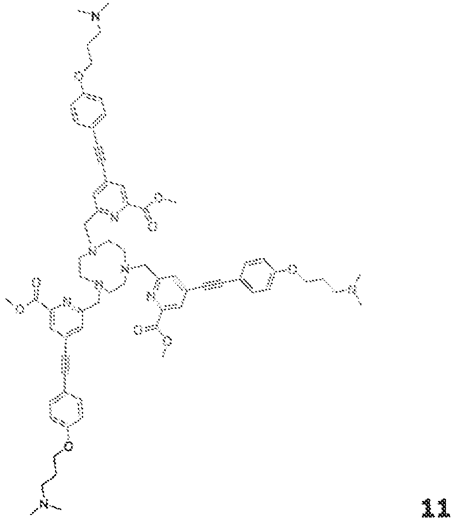

Dans un tube de Schlenk, le dérivé **3** (64mg, 6,76.10⁻⁵mol, 1,0eq) est solubilisé dans un mélange DMF/Et₃N (2mL, 1/1, v/v). La solution est dégazée sous argon pendant 30min. A cette solution sont ensuite ajoutés une solution de **10b** (55mg, 2,70.10⁻⁴ mol, 4,0eq) solubilisé dans du DMF (1 mL) préalablement dégazé sous argon, puis le PdCl₂(PPh₃)₂ (4mg, 5,68.10⁻⁶ mmol, 0,03eq). Le mélange réactionnel est agité sous argon pendant 48h à 80°C. A la fin de la réaction, les solvants sont évaporés sous pression réduite. Du CH₂Cl₂ est ajouté au brut réactionnel et la phase organique est lavée avec une solution saturée en Na₂CO₃, puis à l'eau. La phase organique est ensuite séchée sur Na₂SO₄, filtrée puis évaporée sous pression réduite. Le brut réactionnel est purifié par des précipitations successives dans un mélange AcOEt/Pentane pour mener au composé désiré sous la forme d'une huile orange (52 mg, 65%).
**RMN ¹H (500 MHz, CDCl₃) δ:** 8.03 (s, 3H), 7.85 (s, 3H), 7.45 (d, 6H, *J*= 8.8Hz), 6.88 (d, 6H, *J*= 8.8Hz), 4.03 (t, 6H, *J*=6,2 Hz), 3.96 (m,15 H), 2.96 (brs, 12H), 2.45 (t, 6H, *J*= 6,4 Hz), 2.26 (s, 18H), 1,97 (m, 6H).
RMN **¹³C (125,76 MHz, CDCl₃) δ**: 165,73, 161,57,160,12, 147,47, 133,76, 133,60, 128;74, 127,95, 125,60, 114,87, 113,93, 95,56, 85,65, 66,47? 64,47? 56,40, 56,16, 53,13, 45,59, 45,41, 27,49.
**HRMS (ESI** ⁺): (Calc pour C₆₉H₈₄N₉O₉ : 394,2142 ) ; mesurée : [M+3H]³⁺ : m/z= 394,2125

### i) Préparation du composé 12:

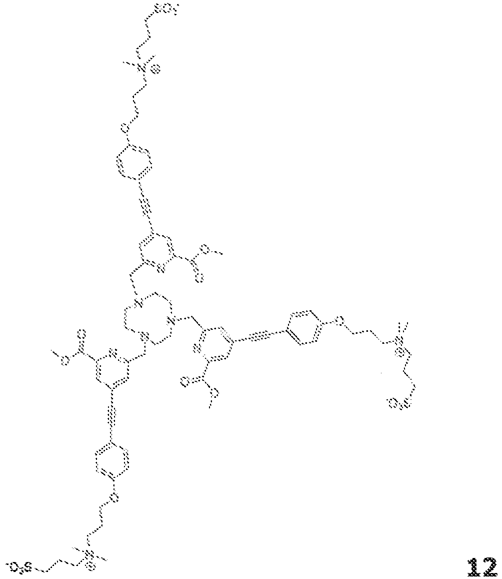

Le composé **11** (36mg, 3,05.10⁻⁵mol, 1,0eq) est solubilisé dans 1mL d'acétone. La 1,3-propanesultone est ajoutée et le mélange réactionnel est agité sous argon à température ambiante pendant 16h. A la fin de la réaction, le solvant est évaporé et le résidu est trituré dans du CH₂Cl₂. Le produit est obtenu sous la forme d'une huile orange (40 mg, 85%).
RMN ¹H (200 MHz, CDCl₃) δ**:** 7,83 (s, 3H), 7,67 (s, 3H), 7,38 (d, 6H, *J*= 8,9 Hz), 6,93 (d, 6H, *J*=8,9 Hz), 4,26 (brs, 4H), 4,16-4,10 (m, 8H), 3,92 (s, 9H), 3,69-3,48 (m, 16H), 3,17 (brs, 12H), 2,94-2,90 (m, 6H), 2,37-2,13 (m, 6H)

### j) Préparation du complexe 13, Eu2

Le composé **12** (25 mg, 1,65.10⁻⁵ mol, 1,0 eq) est solubilisé dans un mélange MeOH/H₂O (4 mL, 1/1, v/v). A cette solution est ajoutée une solution aqueuse de NaOH (1M) (4 mL) et le mélange réactionnel est agité sous argon pendant une heure. A la fin de la réaction une solution aqueuse d'HCl est ajoutée jusqu'à pH=2. A ce mélange sont ensuite ajoutés une solution de Na₂CO₃ (8,0 eq) ainsi que le sel d'EuCl₃.6H₂O (3.0 eq). Le mélange réactionnel est agité sous argon à 50°C pendant 16h. A la fin de la réaction, les solvants sont évaporés sous pression réduite et le complexe est purifié par dialyse dans H₂O pendant 24h. Le produit est obtenu sous la forme d'une poudre blanche.
**HRMS (ESI ⁺)** : (Calc pour C₇₅H₉₀EuN₉Na₄O₁₈S₃: 436.3593 ); mesurée : [M+4Na]⁴⁺ : m/z= 436,3589

### k) Préparation du composé 15

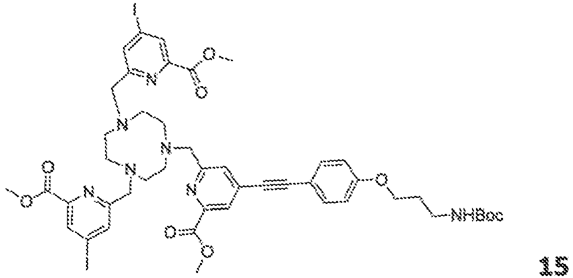

Le composé **6** (86 mg, 1,28.10⁻⁴mol, 1,0 eq) est solubilisé dans 4 mL d'ACN. A cette solution est ajouté sous argon le composé **14** (86 mg, 1,66.10⁻⁴mol, 1,3 eq) et le Na₂CO₃ (40 mg, 3,84.10⁻⁴ mol, 3,0 eq). Le mélange réactionnel est agité sous argon à 50°C pendant 24h. A la fin de laréaction, les solvants sont évaporés et le brut est purifié par colonne chromatographique sur Al₂O₃ (Eluant : CH₂Cl₂/MeOH de 0 à 5% de MeOH). Le produit est obtenu sous la forme d'une huile jaune (m= 60 mg, **42%)**
RMN ¹H (300 MHz**,** CDCl₃) δ**:** 8,07-8,06 (m, 2H), 7,98 (s, 1H), 7,79 (s, 1H), 7,62 (s, 1H), 7,20 (d, 2H, *J*= 8,5Hz), 6,62 (d, 2H, *J*= 8,5 Hz), 3,81-3,76 (m, 4H), 3,73-3,64 (m, 13H), 3,09-3,03 (m, 4H), 2,74-2,62 (m, 9H), 1,73 (t, 2H, *J*= 6,37 Hz), 1,17 (s, 9H).

### Exemple 2 : Complexe de Terbium de formule (IV)

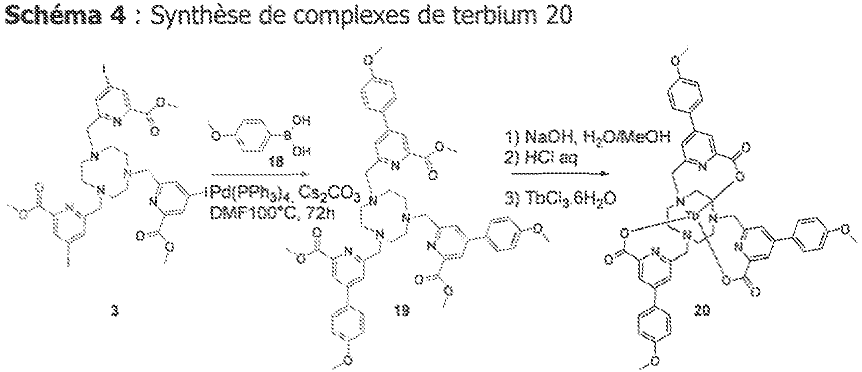

De la même façon, l'intermédiaire **3** peut réagir dans des conditions de couplage de Suzuki en présence de l'acide boronique **18** pour mener au ligand **19.** Les esters méthyliques sont ensuite hydrolysés en milieu basique et le composé réagit *in situ* en présence de sel de Tb(III) pour conduire à la formation du complexe **20.**

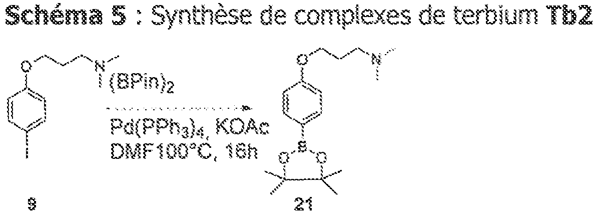

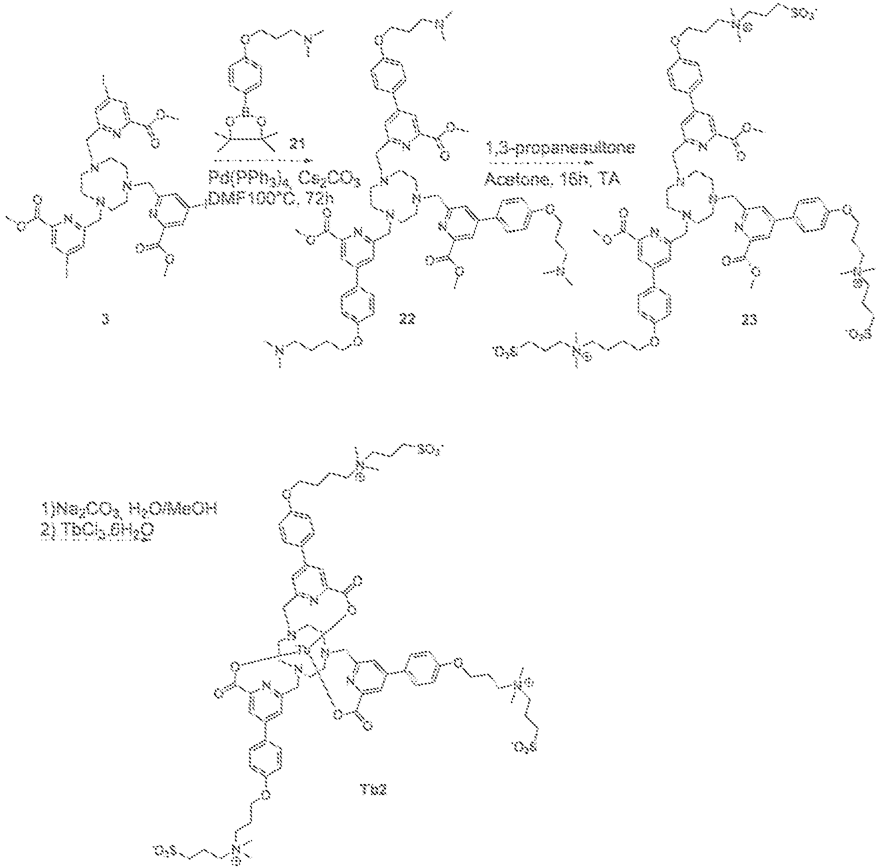

Le complexe **Tb2** porteur de fonctions bétaïnes peut être obtenu via la même voie de synthèse que son analogue **20.** L'intermédiaire **3** peut réagir via une réaction de Suzuki avec l'antenne correspondante **21.** Le ligand **22** pourra réagir en présence de 1,3-propanesultone pour mener au ligand **23.** Comme précédemment le complexe sera formé *in situ* en présence du sel de Tb(III) correspondant pour mener au complexe **Tb2.**

### a) Préparation du composé 19 :

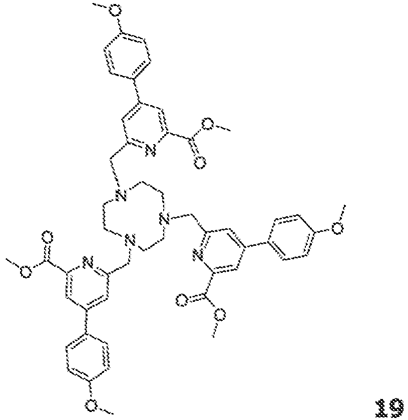

Dans un tube de Schlenk, l'intermédiaire 3 (150mg, 1,57.10⁻⁴ mol, 1,0 eq) et l'acide boronique 18 (79 mg, 5,18.10⁻⁴mol, 3,3eq) sont solubilisés dans du DMF anhydre (4 mL). La solution est dégazée sous argon pendant 30min. A cette solution sont ensuite ajoutés le Cs₂CO₃ (215 mg, 6,59.10⁻⁴mol, 4,2eq) et le Pd(PPh₃)₄ (10mg, 9,42.10⁻⁶mmol, 0,06eq). Le mélange réactionnel est agité sous argon pendant 72h à 100°C. A la fin de la réaction, le Cs₂CO₃ est filtré sous pression réduite et rincé plusieurs fois avec du CH₂Cl₂. Le filtrat est évaporé sous pression réduite et dilué dans CH₂Cl₂. La phase organique est lavée avec une solution de NaCl saturée, puis à l'eau. La phase organique est ensuite séchée sur Na₂SO₄, filtrée puis évaporée sous pression réduite. Le brut réactionnel est purifié par des précipitations successives dans un mélange AcOEt/Pentane pour mener au composé désiré sous la forme d'une huile orange (58 mg, 41%).
**RMN ¹H (200 MHz, CDCl₃) δ:** 8.19 (s, 3H), 8.02 (s, 3H), 7.58 (d, 6H, *J*= 8.8Hz), 6.94 (d, 6H, *J*= 8.9Hz), 4.02 (s, 6H), 3.99 (s, 9H), 3.85 (s, 9H), 3.02 (brs, 12H).
**RMN ¹³C (50.32 MHz, CDCl₃) δ:** 166.33, 161.79, 161.03, 149.37, 147.95, 129.60, 128.35, 123.33, 121.21, 114.81, 65.05, 56.48, 55.59, 29.90.
**HRMS (ESI ⁺):** (Calc pour C₅₁H₅₅N₆O₉ : 895,4032) ; mesurée : [MH]⁺ : m/z= 895,4025.

### b) Préparation du complexe Tb2

Le complexe **Tb2** est préparé de manière analogue au complexe **Eu2,** en utilisant le protocole décrit à l'exemple 1, paragraphe f).
**HRMS (ESI ⁺):** (Calc pour C₄₈H₄₅N₆Na₂O₉Tb : 527,1144); mesurée: [M+2Na]²⁺ : m/z= 527,1143

### B. EVALUATION DES PROPRIETES SPECTROSCOPIQUES DES COMPLEXES

### a) Propriétés spectroscopiques du complexe Eu2 dans l'eau.

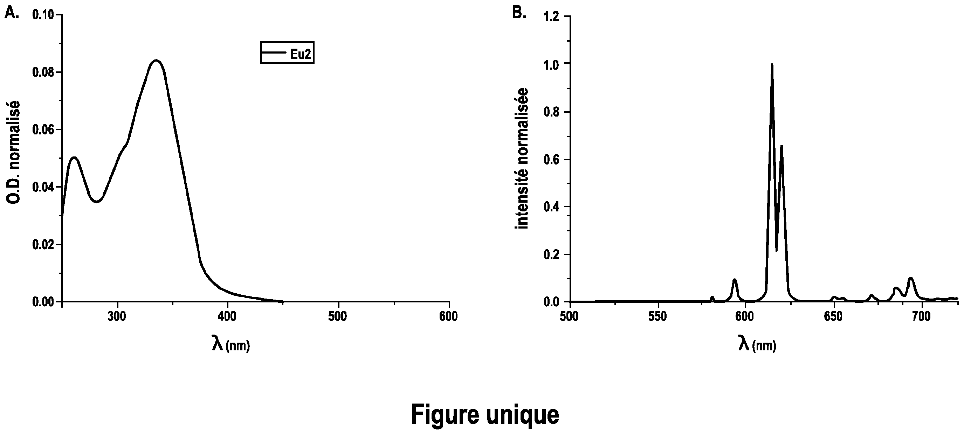

Le complexe **Eu2** présente une bonne solubilité dans l'eau de l'ordre de 10⁻⁴-10⁻⁵ mol.L⁻¹), ainsi qu'une excellente stabilité dans l'eau jusqu'à des dilutions de l'ordre de 10⁻⁷ mol.L⁻¹. La **Figure unique** présente A. le spectre d'absorption du complexe **Eu2** dans l'eau ; B. le spectre d'émission du complexe **Eu2** enregistré dans l'eau (λ_{excit} = 310nm).

Le complexe **Eu2** présente des propriétés spectroscopiques remarquables dans l'eau λₘₐₓ = 335 nm, ε = 50000 L.mol⁻¹.cm⁻¹; Φ = 0,18 et τ = 0,8 ms, ainsi qu'une section efficace à deux photons significative. Sa brillance à 337 nm est donc estimée à 9000 L.mol⁻¹.cm⁻¹, dans le même ordre de grandeur que ses homologues fonctionnalisés par les fragments PEG décrits dans la demande WO 2013/011236. Ces résultats montrent que l'introduction des fonctions bétaïnes ne nuit pas aux propriétés spectroscopiques du complexe.

Dans le cas des autres complexes synthétisés, on constate également que l'introduction de bétaïne permet d'améliorer la solubilité des complexes (en comparaison aux complexes analogues sans bétaïne) et ne modifie pas de manière significative les propriétés luminescentes du complexe.

### C. ETUDE EN MILIEU CELLULAIRE

Le composé **Eu2** a permis la réalisation d'images de cellules fixées par microscopie mono-ou biphotonique. Des cellules cancéreuses T24 (ligne de cellule de cancer de la vessie ATCC, HTB-4, voir A. Picot, A. D'Aléo, P.L Baldeck, A. Grichine, A. Duperray, C. Andraud, O. Maury J. Am. Hem. Soc. 2008, 130, 1532), ont été fixées avec du méthanol et ont été mises en présence d'une solution du complexe (concentration finale dans le milieu = 10⁻⁵ mol.L⁻¹). Les images des cellules ont été enregistrées sur un microscope confocal Zeiss fonctionnant en régime mono ou biphotonique.

Il a été observé que le complexe **Eu2** marque la cellule de façon réversible. En effet, après lavage, le complexe disparait de la cellule indiquant clairement que la fonctionnalisation avec des bétaïnes ne provoque pas une accumulation irréversible dans les tissus lipophiles, contrairement à ce qui est observé lorsque des groupements PEG sont utilisés. Ceci montre clairement que la présence des trois groupes bétaïne permet d'éviter les interactions non spécifiques avec les parties lipophiles de la cellule.

## Revendications

1. Complexes de lanthanide luminescents, comportant un agent chélatant, formé d'un macrocycle à structure 1,4,7-triazacyclononane complexant un ion lanthanide Ln³⁺choisi parmi Eu³⁺, Sm³⁺, Tb³⁺ ou Dy³⁺, **caractérisés en ce que** l'agent chélatant est formé d'un macrocycle substitué par au moins deux groupes bétaïne.

2. Complexes de lanthanide selon la revendication 1 **caractérisés en ce que** l'agent chélatant est substitué par au plus 12 groupes bétaïne, par exemple 2, 3, 4, 5, 6, 7, 8, 9, 10 ou 11 groupes bétaïne.

3. Complexes de lanthanide selon l'une des revendications précédentes **caractérisés en ce que** l'agent chélatant est substitué par au moins une, et en particulier une seule, fonction réactive permettant son couplage par liaison covalente à une biomolécule, la dite fonction réactive étant, de préférence, choisie parmi - COOH, -NH₂, un acrylamide, une amine activée, un ester activé, un aldhéhyde, un halogénure d'alkyle, un anhydride, une aniline, un azide, une aziridine, un acide carboxylique, un diazoalcane, un haloacétamide, une halotriazine, une hydrazine, un imido ester, un isocyanate, un isothiocyanate, un maléimide, un halogénure de sulfonyle, un thiol, une cétone, une amine, un halogénure d'acide, un ester d'hydroxysuccinimidyle, un ester de succinimidyle, un ester d'hydroxysulfosuccinimidyle, un azidonitrophényle, un azidophényle, un 3-(2-pyridyl dithio)-propionamide, un glyoxal, une triazine, un groupe acétylénique, et les groupes de formule : 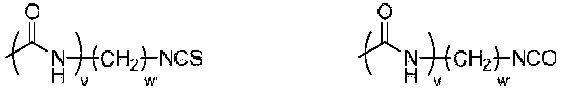 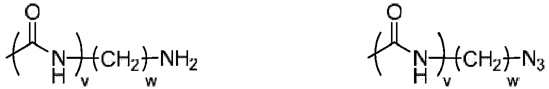 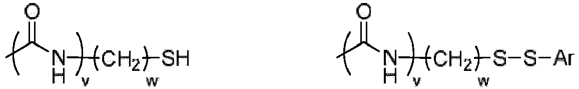 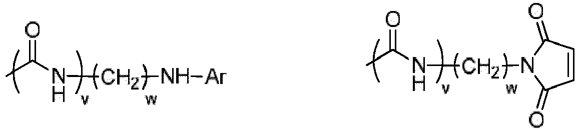 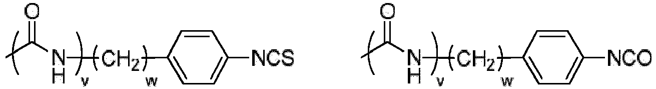 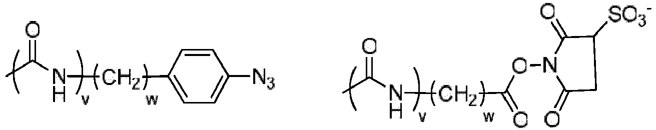 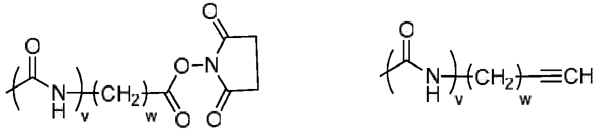 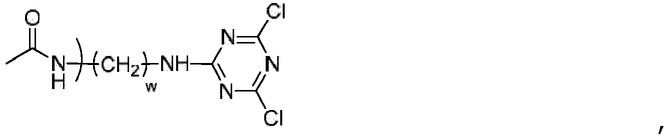 dans lesquels w est un entier appartenant à la gamme allant de 0 à 8 et v est égal à 0 ou 1, et Ar est un hétérocycle à 5 ou 6 chaînons saturé ou insaturé, comprenant 1 à 3 hétéroatomes, éventuellement substitué par un atome d'halogène ;
les fonctions réactives choisies parmi -COOH, -NH₂, les esters de succinimidyle, les haloacétamides, les azides, les hydrazines, les isocyanates et les maléimides étant préférées.

4. Complexes de lanthanide selon l'une des revendications précédentes choisis parmi les complexes de lanthanide de formule (IV) : 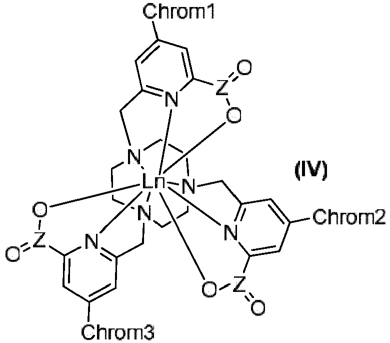 dans lesquelles :
- Ln est un lanthanide choisi parmi Eu, Sm, Tb ou Dy,
- Z représente -C- ou -PR₃-,
- R₃ représente un groupe phényle, benzyle, méthyle, éthyle, propyle, n-butyle, sec-butyle, iso-butyle ou tert-butyle, et de préférence un groupe phényle ou méthyle,
- Chrom1, Chrom2 et Chrom3, identiques ou différents, sont choisis parmi les groupements : 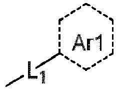
dans lesquels :
- L₁ représente une liaison directe, -C=C- ou -C≡C-,
- Ar₁ représente un groupe aromatique choisi parmi les groupes phényle, thiophényle, furanyle, pyrrolyle, imidazolyle ou triazolyle, substitué par s groupes R₀ identiques ou différents,
- s est égal à 1, 2 ou 3, et
- R₀ est choisi parmi les groupes alkyle comprenant de 1 à 10, et de préférence de 1 à 6, atomes de carbone ; les groupes alkyle comprenant de 1 à 10 atomes de carbone porteurs d'au moins une fonction bétaïne et/ou d'une fonction réactive ; et les groupements électrodonneurs, notamment O-donneurs, S-donneurs, NHCO-donneurs, SCO-donneurs, NHCS-donneurs, et SCS-donneurs, lesdits groupes électrodonneurs pouvant être porteurs ou non d'un ou plusieurs groupes bétaïne, et/ou d'une fonction réactive,
étant entendu que lorsque tous les groupes Ar1 représentent un groupe phényle, au moins l'un de ces groupes phényle, de préférence deux, voire trois de ces groupes, est(sont) substitué(s) par au moins un groupe R₀ comportant un groupement électrodonneur ;
**caractérisés en ce qu'**au moins deux des groupements Chrom1, Chrom2 et Chrom3 sont substitués par au moins un groupe R₀ porteur d'au moins un groupe bétaïne.

5. Complexes de lanthanide selon la revendication 4 **caractérisés en ce que** Chrom1, Chrom2 et Chrom3 sont définis comme suit :
a. Soit Chrom1, Chrom2 et Chrom3, identiques ou différents, sont choisis parmi les groupements : 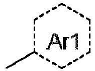 avec Ar1 tel que défini à la revendication 5,
b. Soit Chrom1, Chrom2 et Chrom3, identiques ou différents, sont choisis parmi les groupements : 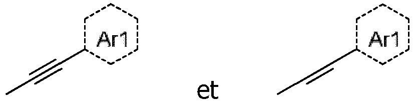 avec Ar1 qui représente un groupe phényle, thiophényle, furanyle, pyrrolyle ou imidazolyle substitué par s groupes R₀ identiques ou différents, s et R₀ étant tels que définis à la revendication 5.

6. Complexes de lanthanide selon la revendication 4 ou 5 **caractérisés en ce que** les substituants R₀, identiques ou différents, sont choisis parmi :
- -L₂-Alk, - L₂- L₃-Q₁ et -L₂-L₃-Q₂ ;
- les groupements NHCO-donneurs choisis parmi : -NHCO(OAlk),-NHCO(NHAlk), -NHCO(NAlklAlk2), -NHCO(SAlk),
- les groupements SCO-donneurs choisis parmi : -SCO(OAlk), -SCO(NHAlk),-SCO(NAlklA1k2), -SCO(SAlk),
- les groupements NHCS-donneurs choisis parmi : -NHCS(OAlk), -NHCS(NHAlk), -NHCS(NAlk1Alk2), et
- les groupements SCS-donneurs choisis parmi : -SCS(OAlk), -SCS(NHAlk),-SCS(NAlk1Alk2), -SCS(SAlk),
- Alk, Alk1 et Alk2, identiques ou différents, sont des groupes alkyle comprenant de 1 à 10 atomes de carbone, éventuellement substitués par au moins un groupe bétaïne,
- Q₁ représente un groupe bétaïne ou un groupement ramifié porteur d'au moins deux groupes bétaïne,
- L₂ est une liaison directe, -O-, -S-, -NHCO-, -SCO-, -NHCS- ou -SCS-
- L₃ est un bras de liaison, et
- Q₂ est un groupe réactif susceptible de permettre la liaison covalente avec une molécule d'intérêt à marquer,
étant entendu qu'au moins deux des substituants R₀ présents sont porteurs d'au moins un groupe bétaïne, de manière à ce qu'au moins deux des groupements Chrom1, Chrom2 et Chrom3 soient substitués par au moins un groupe R₀ porteur d'au moins un groupe bétaïne.

7. Complexes de lanthanide selon la revendication 4, 5 ou 6 **caractérisés en ce que** Chrom1 = Chrom2 = Chrom3.

8. Complexes de lanthanide selon la revendication 4, 5 ou 6 **caractérisés en ce que** Chrom1 = Chrom2 et sont substitués par au moins un groupe R₀ porteur d'au moins un groupe bétaïne et Chrom3 est substitué par au moins un groupement R₀ porteur d'une fonction -L₂-L₃-Q₂, tel que défini à la revendication 7, avec de préférence Q₂ qui représente un groupe choisi parmi -COOH, -NH₂, un acrylamide, une amine activée, un ester activé, un aldhéhyde, un halogénure d'alkyle, un anhydride, une aniline, un azide, une aziridine, un acide carboxylique, un diazoalcane, un haloacétamide, une halotriazine, une hydrazine, un imido ester, un isocyanate, un isothiocyanate, un maléimide, un halogénure de sulfonyle, un thiol, une cétone, une amine, un halogénure d'acide, un ester d'hydroxysuccinimidyle, un ester de succinimidyle, un ester d'hydroxysulfosuccinimidyle, un azidonitrophényle, un azidophényle, un 3-(2-pyridyl dithio)-propionamide, un glyoxal, une triazine, un groupe acétylénique, et les groupes de formule : 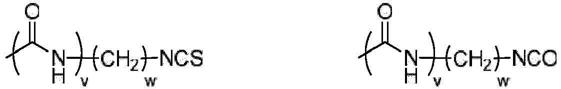 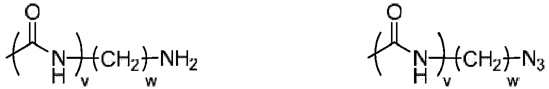 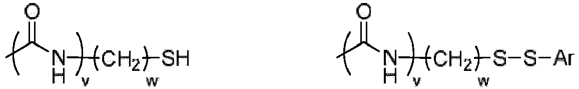 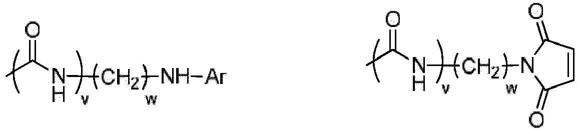 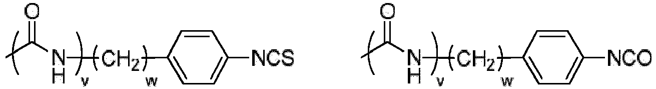 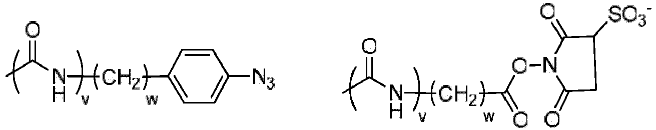 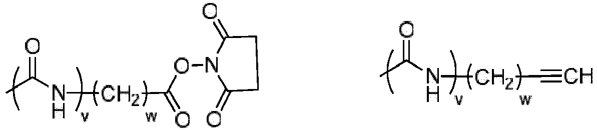 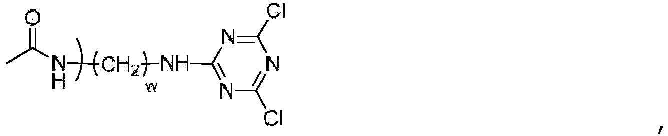 dans lesquels w est un entier appartenant à la gamme allant de 0 à 8 et v est égal à 0 ou 1, et Ar est un hétérocycle à 5 ou 6 chaînons saturé ou insaturé, comprenant 1 à 3 hétéroatomes, éventuellement substitué par un atome d'halogène ;
Q₂ étant de préférence choisi parmi -COOH, -NH₂, les esters de succinimidyle, les haloacétamides, les hydrazines, les isocyanates et les maléimides étant préférées.

9. Complexes de lanthanide selon l'une des revendications 4 à 8 **caractérisés en ce que** L₁ représente une liaison directe ou -C≡C-, et les groupes Ar1, identiques ou différents, représentent chacun un groupe phényle, substitué par s groupes R₀ identiques ou différents, avec s et R₀ qui sont tels que définis à la revendication 5, 7, 8 ou 9.

10. Complexes de lanthanide selon l'une des revendications 4 à 9 **caractérisés en ce que** dans tous les groupes Ar1, s est égal à 1.

11. Complexes de lanthanide selon l'une des revendications 4 à 10 **caractérisés en ce que** les groupes Ar1, identiques ou différents, représentent chacun un groupe phényle choisi parmi les groupements : 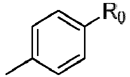 avec R₀ tel que défini à la revendication 5, 7, 8 ou 9.

12. Complexes de lanthanide selon la revendication 6 ou 8 **caractérisés en ce que** L₃ représente une liaison covalente, un groupe alkylène de 1 à 12 atomes de carbone, comprenant éventuellement une ou plusieurs double ou triple liaison ; un groupe cycloalkylène de 5 à 8 atomes de carbone, un groupe arylène de 6 à 14 atomes de carbone ; ou un enchainement de un ou plusieurs groupes alkylène de 1 à 12 atomes de carbone, cycloalkylène de 5 à 8 atomes de carbone et/ou arylène de 6 à 14 atomes de carbone ; lesdits groupes alkylène, cycloalkylène ou arylène pouvant ou non comprendre un ou plusieurs hétéroatomes tels que l'oxygène, l'azote, le soufre, le phosphore ou un ou plusieurs carbamoyle ou carboxamido et/ou pouvant être non substitués ou substitués par un ou plusieurs groupe alkyle de 1 à 8 atomes de carbone, aryle de 6 à 14 atome de carbone, sulfonate ou oxo ; les bras de liaison suivants étant préférés : 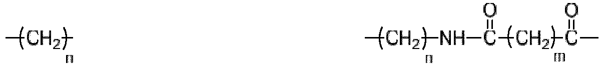 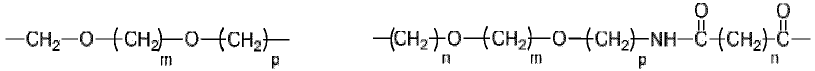 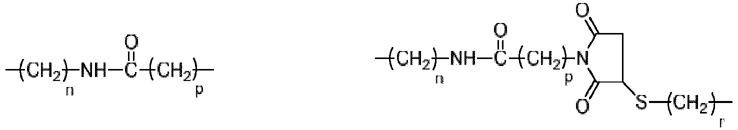 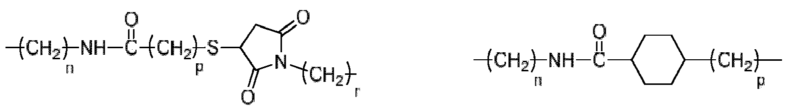 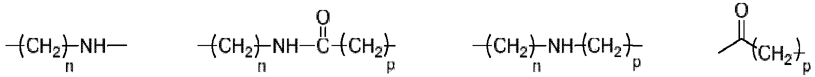 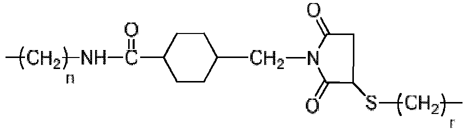
n est égal à 1, 2, 3, 4, 5 ou 6,
m, p et r, identiques ou différents, sont égaux à 1, 2 ou 3,

13. Complexes de lanthanide selon l'une des revendications 4 à 12 **caractérisés en ce que** :
- soit Ln³⁺= Eu³⁺ ou Sm³⁺ et L₁ représente -C≡C- ;
- soit Ln³⁺= Eu³⁺, Sm³⁺, Tb³⁺ ou Dy³⁺ et, de préférence Tb³⁺ ou Dy³⁺, et L₁ représente une liaison directe.

14. Complexes de lanthanide selon l'une des revendications 1 à 13 **caractérisés en ce que** les groupes bétaïne sont choisis parmi les groupements zwittérioniques associant un cation ammonium, ou iminium aromatique, généralement pyridinium, imidazollium, et un groupement anionique de type sulfonate, phosphonate ou carboxylate, de préférence sulfonate, ledit cation et le dit anion étant espacés par au moins un chainon CH₂, et de préférence par une chaine alkyle bivalente comprenant de 1 à 4, voire de 1 à 6, atomes de carbone.

15. Complexes de lanthanide selon l'une des revendications 1 à 14 **caractérisés en ce que** les groupes bétaïne sont choisis parmi : 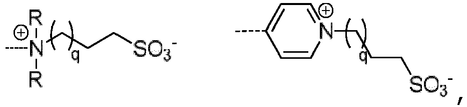 avec R qui représente un groupe alkyle de 1 à 6 atomes de carbone, et de préférence un méthyle ou éthyle, et q qui est égal à 1, 2, 3, 4, 5 ou 6, et de préférence qui est égal à 1 ou 2, le groupe -N(CH₃)₂⁺-(CH₂)₃-SO₃⁻ étant préféré.

16. Complexes de lanthanide selon les revendications 1 à 15 **caractérisés en ce que** les bétaïnes sont des groupes hydrosolubilisants assurant la solubilisation des complexes en milieu aqueux.

17. Agent chélatant de formule (II) : 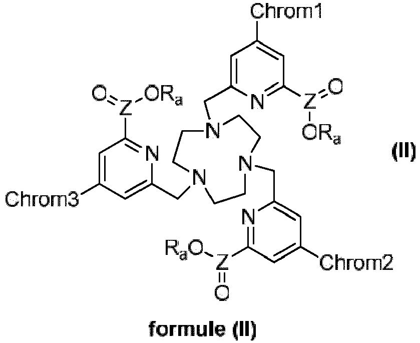 avec Ra qui est un groupe protecteur des fonctions acides tel qu'un alkyle, du type méthyle, et Z, Chrom1, Chrom2 et Chrom3, identiques ou différents, qui sont tels que définis aux revendications 4 à 11, 12 ou 14.

## Patentansprüche

1. Lumineszierende Lanthanidkomplexe, aufweisend einen Chelatbildner, gebildet von einem Strukturmakroring 1,4,7-Triazacyclononan, komplexierend ein Lanthanidion Ln³⁺, ausgewählt aus Eu³⁺, Sm³⁺, Tb³⁺ oder Dy³⁺, **dadurch gekennzeichnet, dass** der Chelatbildner von einem Makroring gebildet ist, der durch mindestens zwei Betaingruppen substituiert ist.

2. Lanthanidkomplexe nach Anspruch 1, **dadurch gekennzeichnet, dass** der Chelatbildner durch höchsten 12 Betaingruppen, beispielsweise 2, 3, 4, 5, 6, 7, 8, 9, 10 oder 11 Betaingruppen, substituiert ist.

3. Lanthanidkomplexe nach einem der vorangehenden Ansprüche, **dadurch gekennzeichnet, dass** der Chelatbildner durch mindestens eine und insbesondere eine einzige reaktive Funktion substituiert ist, die seine Kopplung durch kovalente Bindung an ein Biomolekül erlaubt, wobei die reaktive Funktion vorzugsweise ausgewählt ist aus -COOH, -NH₂, einem Acrylamid, einem aktivierten Amin, einem aktivierten Ester, einem Aldehyd, einem Alkylhalogenid, einem Anhydrid, einem Anilin, einem Azid, einem Aziridin, einer Carbonsäure, einem Diazoalcan, einem Haloacetamid, einem Halotriazin, einem Hydrazin, einem Imidoester, einem Isocyanat, einem Isothiocyanat, einem Maleimid, einem Sulfonylhalogenid, einem Thiol, einem Keton, einem Amin, einem Säurehalogenid, einem Hydroxysuccinimidylester, einem Succinimidylester, einem Hydroxysulfosuccinimidylester, einem Azidonitrophenyl, einem Azidophenyl, einem 3-(2-Pyridyldithio)-propionamid, einem Glyoxal, einem Triazin, einer Acetylengruppe und den Gruppen der Formel: 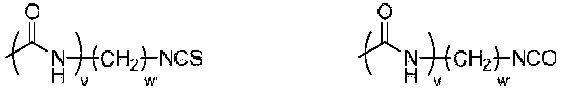 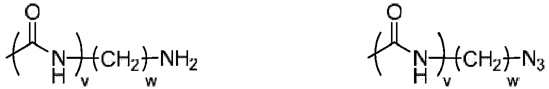 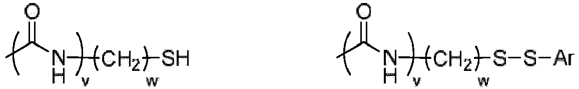 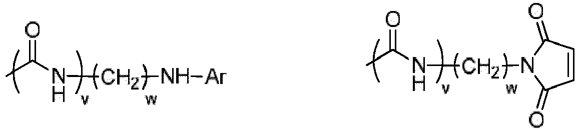 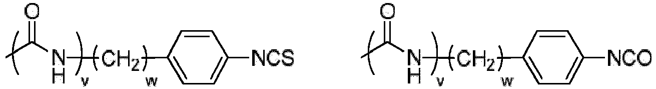 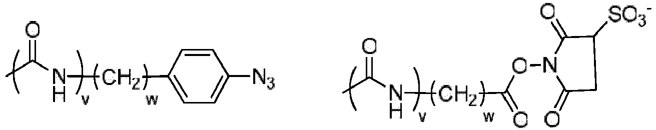 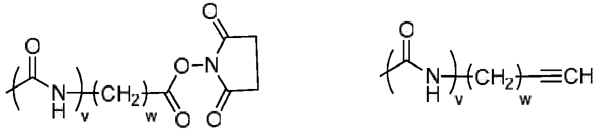 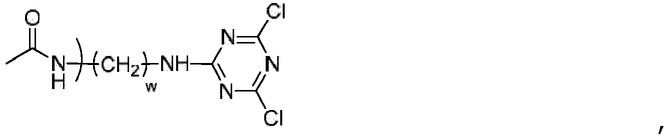 wobei w eine Ganzzahl ist, die zu dem Bereich gehört, der von 0 bis 8 geht, und v gleich 0 oder 1 ist, und Ar ein Heteroring mit 5 oder 6 gesättigten oder ungesättigten Gliedern ist, umfassend 1 bis 3 Heteroatome, eventuell substituiert durch ein Halogenatom;
wobei die reaktiven Funktionen, ausgewählt aus -COOH, -NH₂, den Succinimidylestern, den Haloacetamiden, den Aziden, den Hydrazinen, den Isocyanaten und den Maleimiden, bevorzugt sind.

4. Lanthanidkomplexe nach einem der vorangehenden Ansprüche, ausgewählt aus den Lanthanidkomplexen der Formel (IV) : 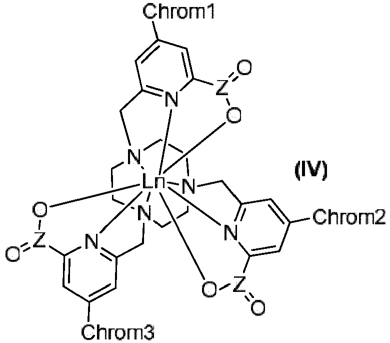 wobei:
- Ln ein Lanthanid ist, ausgewählt aus Eu, Sm, Tb oder Dy,
- Z -C- oder -PR₃- darstellt,
- R₃ eine Phenyl-, Benzyl-, Methyl-, Ethyl-, Propyl-, n-Butyl-, sec-Butyl-, iso-Butyl- oder tert-Butylgruppe darstellt und vorzugsweise eine Phenyl- oder Methylgruppe,
- Chrom1, Chrom2 und Chrom3, die identisch oder unterschiedlich sind, aus den Gruppen ausgewählt sind: 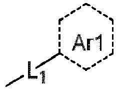
wobei:
- L₁ eine direkte Bindung, -C=C- oder -C≡C- darstellt,
- Ar₁ eine aromatische Gruppe darstellt, ausgewählt aus den Phenyl-, Thiophenyl-, Furanyl-, Pyrrolyl-, Imidazolyl- oder Triazolylgruppen, substituiert durch s Gruppen R₀, die identisch oder unterschiedlich sind,
- s gleich 1, 2 oder 3 ist, und
- R₀ aus den Alkylgruppen ausgewählt ist, umfassend 1 bis 10 und vorzugsweise 1 bis 6 Kohlenstoffatome; den Alkylgruppen, umfassend 1 bis 10 Kohlenstoffatome, die mindestens eine Betainfunktion und/oder einer reaktive Funktion tragen; und den Elektronendonorgruppen, insbesondere O-Donoren, S-Donoren, NHCO-Donoren, SCO-Donoren, NHCS-Donoren und SCS-Donoren, wobei die Elektronendonorgruppen Träger von einer oder mehreren Betaingruppen und/oder von einer reaktiven Funktion sein können oder nicht,
wobei, wenn alle Gruppen Ar1 eine Phenylgruppe darstellen, mindestens eine dieser Phenylgruppen, vorzugsweise zwei, sogar drei dieser Gruppen durch mindestens eine Gruppe R₀ substituiert ist/sind, die eine Elektronendonorgruppe aufweist;
**dadurch gekennzeichnet, dass** mindestens zwei der Gruppen Chrom1, Chrom2 und Chrom3 durch mindestens eine Gruppe R₀ substituiert sind, die Träger von mindestens einer Betaingruppe ist.

5. Lanthanidkomplexe nach Anspruch 4, **dadurch gekennzeichnet, dass** Chrom1, Chrom2 und Chrom3 wie folgt definiert sind:
a. entweder sind Chrom1, Chrom2 und Chrom3, die identisch oder unterschiedlich sind, ausgewählt aus den Gruppen: 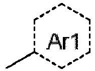 mit Ar1 wie in Anspruch 5 definiert,
b. oder Chrom1, Chrom2 und Chrom3, die identisch oder unterschiedlich sind, sind ausgewählt aus den Gruppen: 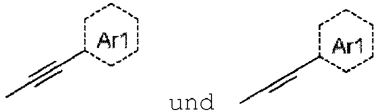 mit Ar1, das eine Phenyl-, Thiophenyl-, Furanyl-, Pyrrolyl- oder Imidazolylgruppe darstellt, substituiert durch s Gruppen R₀, die identisch oder unterschiedlich sind, wobei s und R₀ derart sind, wie in Anspruch 5 definiert.

6. Lanthanidkomplexe nach Anspruch 4 oder 5, **dadurch gekennzeichnet, dass** die Substituenten R₀, die identisch oder unterschiedlich sind, ausgewählt sind aus:
- -L₂-Alk, -L₂-L₃-Q₁ und -L₂-L₃-Q₂;
- den NHCO-Donorgruppen, ausgewählt aus: -NHCO(OAIk), NHCO(NHAIk), -NHCO(NAlk1Alk2), -NHCO(SAlk),
- den SCO-Donorgruppen, ausgewählt aus: -SCO(OAIk), - SCO(NHAlk), -SCO(NAlklAlk2), -SCO(SAlk),
- den NHCS-Donorgruppen, ausgewählt aus: -NHCS(OAIk), - NHCS(NHAlk), -NHCS(NAIk1Alk2) , und
- den SCS-Donorgruppen, ausgewählt aus: -SCS(OAlk), - SCS(NHAlk), -SCS(NAlk1Alk2), -SCS(SAlk),
- wobei Alk, Alk1 und Alk2, die identisch oder unterschiedlich sind, Alkylgruppen sind, umfassend 1 bis 10 Kohlenstoffatome, substituiert eventuell durch mindestens eine Betaingruppe,
- Q₁ eine Betaingruppe oder eine verzweigte Gruppe als Träger von mindestens zwei Betaingruppen darstellt,
- L₂ eine direkte Bindung, -O-, -S-, -NHCO-, -SCO-, -NHCS- oder -SCS- ist,
- L₃ ein Bindungsarm ist, und
- Q₂ eine reaktive Gruppe ist, welche geeignet ist, die kovalente Bindung mit einem zu markierenden Molekül von Interesse zu erlauben,
wobei mindestens zwei der vorhandenen Substituenten R₀ Träger mindestens einer Betaingruppe sind, so dass mindestens zwei der Gruppen Chrom1, Chrom2 und Chrom3 durch mindestens eine Gruppe R₀ als Träger mindestens einer Betaingruppe substituiert sind.

7. Lanthanidkomplexe nach Anspruch 4, 5 oder 6, **dadurch gekennzeichnet, dass** Chrom1 = Chrom2 = Chrom3.

8. Lanthanidkomplexe nach Anspruch 4, 5 oder 6, **dadurch gekennzeichnet, dass** Chrom1 = Chrom2 durch mindestens eine Gruppe R₀ als Träger mindestens einer Betaingruppe substituiert sind und Chrom3 durch mindestens eine Gruppe R₀ als Träger einer Funktion -L₂-L₃-Q₂ substituiert ist, wie in Anspruch 7 definiert, mit vorzugsweise Q₂, das eine Gruppe darstellt, die aus -COOH, -NH₂, einem Acrylamid, einem aktivierten Amin, einem aktivierten Ester, einem Aldhehyd, einem Alkylhalogennid, einem Anhydrid, einem Anilin, einem Azid, einem Aziridin, einer Carbonsäure, einem Diazoaican, einem Haloacetamid, einem Halotriazin, einem Hydrazin, einem Imidoester, einem Isocyanat, einem Isothiocyanat, einem Maleimid, einem Sulfonylhalogenid, einem Thiol, einem Keton, einem Amin, einem Säurehalogenid, einem Hydroxysuccinimidylester, einem Succinimidylester, einem Hydroxysulfosuccinimidylester, einem Azidonitrophenyl, einem Azidophenyl, einem 3-(2-Pyridyldithio)-propionamid, einem Glyoxal, einem Triazin, einer Acetylengruppe ausgewählt ist, und den Gruppen der Formel: 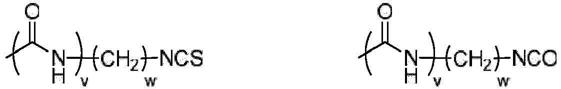 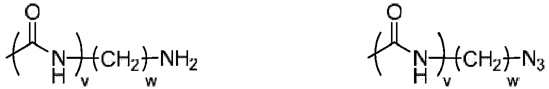 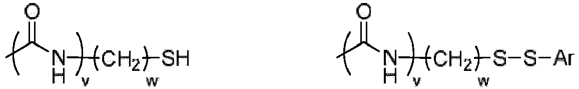 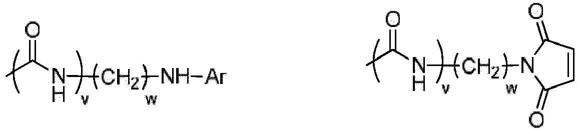 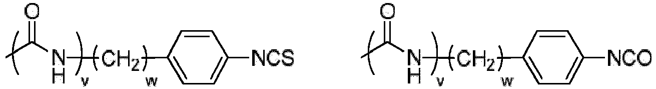 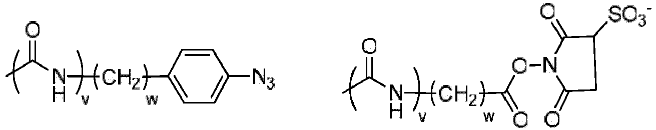 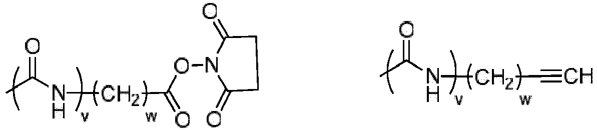 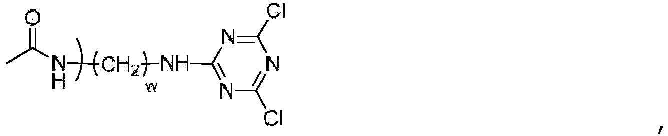
wobei w eine Ganzzahl ist, die zu dem Bereich gehört, der von 0 bis 8 geht, und v gleich 0 oder 1 ist, und Ar ein Heteroring mit 5 oder 6 gesättigten oder ungesättigten Gliedern ist, umfassend 1 bis 3 Heteroatome, eventuell substituiert durch ein Halogenatom;
wobei Q₂, vorzugsweise aus -COOH, -NH₂, den Succinimidylestern, den Haloacetamiden, den Aziden, den Hydrazinen, den Isocyanaten und den Maleimiden ausgewählt, bevorzugt sind.

9. Lanthanidkomplexe nach einem der Ansprüche 4 bis 8, **dadurch gekennzeichnet, dass** L₁ eine direkte Bindung oder -C≡Cdarstellt, und die Gruppen Ar1, die identisch oder unterschiedlich sind, jeweils eine Phenylgruppe darstellen, substituiert durch s Gruppen R₀, die identisch oder unterschiedlich sind, mit s und R₀, die wie im Anspruch 5, 7, 8 oder 9 definiert sind.

10. Lanthanidkomplexe nach einem der Ansprüche 4 bis 9, **dadurch gekennzeichnet, dass** in allen Gruppen Ar1 s gleich 1 ist.

11. Lanthanidkomplexe nach einem der Ansprüche 4 bis 10, **dadurch gekennzeichnet, dass** die Gruppen Ar1, die identisch oder unterschiedlich sind, jeweils eine Phenylgruppe darstellen, ausgewählt aus den Gruppen: 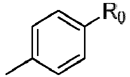 mit R₀ wie in Anspruch 5, 7, 8 oder 9 definiert.

12. Lanthanidkomplexe nach Anspruch 6 oder 8, **dadurch gekennzeichnet, dass** L₃ eine kovalente Bindung, eine Alkylengruppe mit 1 bis 12 Kohlenstoffatomen, umfassend eventuell eine oder mehrere Doppel- oder Dreifachbindungen; eine Cycloalkylengruppe mit 5 bis 8 Kohlenstoffatomen, eine Arylengruppe mit 6 bis 14 Kohlenstoffatomen; oder eine Verkettung von einer oder mehreren Alkylengruppen mit 1 bis 12 Kohlenstoffatomen, Cycloalkylengruppen mit 5 bis 8 Kohlenstoffatome und/oder Arylengruppen mit 6 bis 14 Kohlenstoffatomen darstellt; wobei die Alkylen-, Cycloalkylen- oder Arylengruppen ein oder mehrere Heteroatome wie Sauerstoff, Stickstoff, Schwefel, Phosphor oder ein oder mehrere Carbamoyle oder Carboxamido umfassen können oder nicht und/oder durch eine oder mehrere Alkylgruppen mit 1 bis 8 Kohlenstoffatomen, Arylgruppen mit 6 bis 14 Kohlenstoffatomen, Sulfonat- oder Oxogruppen nicht substituiert oder substituiert sein können; wobei die folgenden Bindungsarme bevorzugt sind: 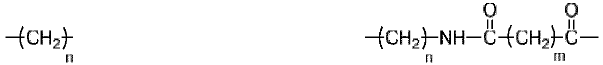 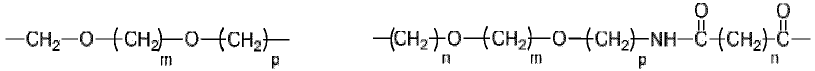 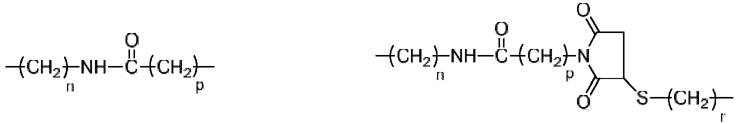 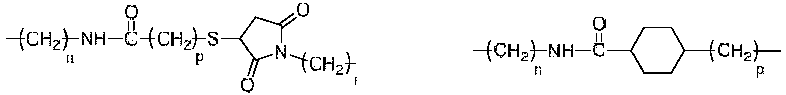 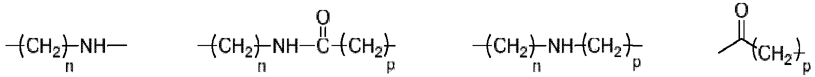 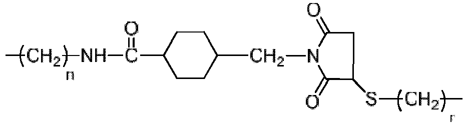
wobei n gleich 1, 2, 3; 4, 5 oder 6 ist,
wobei m, p und r, die identisch oder unterschiedlich sind, gleich 1, 2 oder 3 sind.

13. Lanthanidkomplexe nach einem der Ansprüche 4 bis 12, **dadurch gekennzeichnet, dass**:
- entweder Ln³⁺ = Eu³⁺ oder Sm³⁺ und L₁ stellt -C≡C- dar;
- oder Ln³⁺ = Eu³⁺, Sₘ³⁺, Tb³⁺ oder Dy³⁺ und, vorzugsweise, Tb³⁺ oder Dy³⁺, und L₁ stellt eine direkte Bindung dar.

14. Lanthanidkomplexe nach einem der Ansprüche 1 bis 13, **dadurch gekennzeichnet, dass** die Betaingruppen aus den zwitterionischen Gruppen ausgewählt sind, die ein Ammoniumkation, oder aromatisches Iminium, im Allgemeinen Pyridinium, Imidazollium, und eine anionische Gruppe vom Typ Sulfonat, Phosphonat oder Carboxylat, vorzugsweise Sulfonat, kombinieren, wobei das Kation und das Anion durch mindestens eine Kette CH₂ und vorzugsweise durch eine zweiwertige Alkylkette, umfassend 1 bis 4, sogar 1 bis 6 Kohlenstoffatome, beabstandet sind.

15. Lanthanidkomplexe nach einem der Ansprüche 1 bis 14, **dadurch gekennzeichnet, dass** die Betaingruppen ausgewählt sind aus: 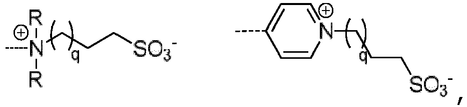 mit R, das eine Alkylgruppe mit 1 bis 6 Kohlenstoffatomen darstellt, und vorzugsweise ein Methyl oder Ethyl, und q, das gleich 1, 2, 3, 4, 5 oder 6 ist, und das vorzugsweise gleich 1 oder 2 ist, wobei die Gruppe -N(CH₃)₂₊-(CH₂)₃-SO-₃⁻ bevorzugt ist.

16. Lanthanidkomplexe nach den Ansprüchen 1 bis 15, **dadurch gekennzeichnet, dass** die Betaine hydrosolubilisierende Gruppen sind, welche die Solubilisierung der Komplexe in wässrigem Milieu sichern.

17. Chelatbildner der Formel (II): 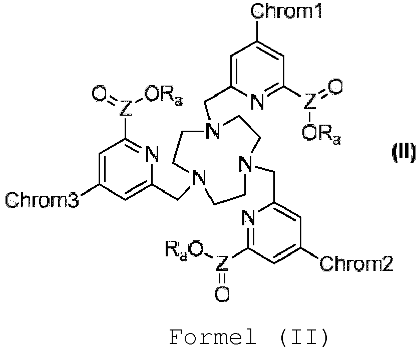 mit Ra, das eine Schutzgruppe der Säurefunktionen ist wie ein Alkyl vom Typ Methyl, und Z, Chrom1, Chrom2 und Chrom3, die identisch oder unterschiedlich sind, die wie in den Ansprüchen 4 bis 11, 12 oder 14 definiert sind.

## Claims

1. Luminescent lanthanide complexes including a chelating agent, formed with a macrocycle with a 1,4,7-triazacyclononane structure complexing a lanthanide ion Ln³⁺ chosen among Eu³⁺, Sm³⁺, Tb³⁺ or Dy³⁺, **characterized in that** the chelating agent is formed with a macrocycle substituted with at least two betaine groups.

2. Lanthanide complexes according to claim 1, **characterized in that** the chelating agent is substituted with at most 12 betaine groups, for example 2, 3, 4, 5, 6, 7, 8, 9, 10 or 11 betaine groups.

3. Lanthanide complexes according to one of the preceding claims, **characterized in that** the chelating agent is substituted with at least one, and in particular only one, reactive function allowing its coupling through a covalent bond with a biomolecule, said reactive function being preferably selected among -COOH, -NH₂, an acrylamide, an activated amine, an activated ester, an aldehyde, an alkyl halide, an anhydride, an aniline, an azide, an aziridine, a carboxylic acid, a diazoalkane, a haloacetamide, a halotriazine, a hydrazine, an imido ester, an isocyanate, an isothiocyanate, a maleimide, a sulfonyl halide, a thiol, a ketone, an amine, an acid halide, a hydroxysuccinimidyl ester, a succinimidyl ester, a hydroxysulfosuccinimidyl ester, an azidonitrophenyl, an azidophenyl, a 3-(2-pyridyl dithio)-propionamide, a glyoxal, a triazine, an acetylenic group, and the groups of formula: 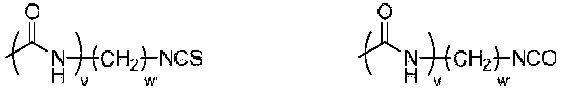 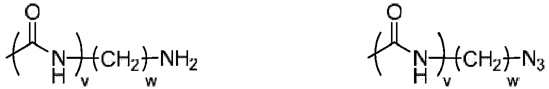 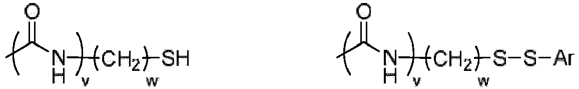 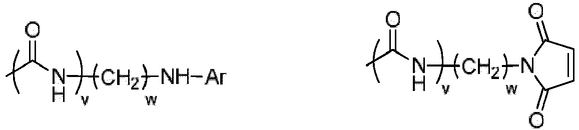 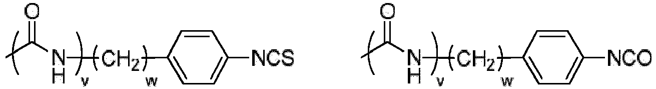 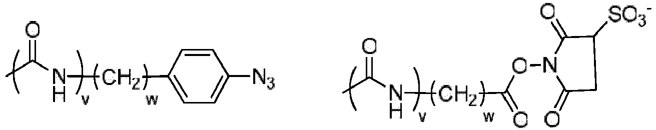 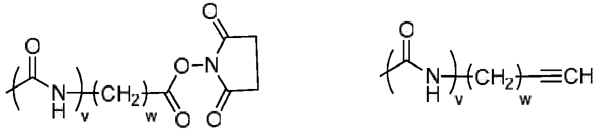 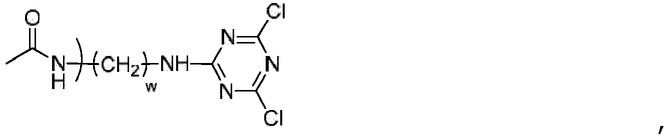 wherein w is an integer belonging to the range from 0 to 8 and v is equal to 0 or 1, and Ar is a saturated or unsaturated 5- or 6-membered heterocycle comprising from 1 to 3 heteroatoms, optionally substituted with a halogen atom;
the reactive functions selected from -COOH, -NH₂, succinimidyl esters, haloacetamides, azides, hydrazines, isocyanates and maleimides being preferred.

4. Lanthanide complexes according to one of the preceding claims, selected from the lanthanide complexes of formula (IV): 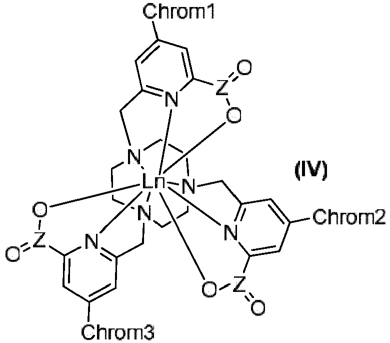 wherein:
- Ln is a lanthanide chosen among Eu, Sm, Tb or Dy,
- Z represents -C- or -PR₃-,
- R₃ represents a phenyl, benzyl, methyl, ethyl, propyl, n-butyl, sec-butyl, iso-butyl or tert-butyl group, and preferably a phenyl or methyl group,
- Chrom1, Chrom2 and Chrom3, either identical or different, are selected from the groups: 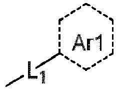
wherein:
- L₁ represents a direct bond, -C=C- or -C≡C-,
- Ar1 represents an aromatic group selected from phenyl, thiophenyl, furanyl, pyrrolyl, imidazolyl or triazolyl groups, substituted with s groups R₀ identical or different,
- s is equal to 1, 2 or 3, and
- R₀ is selected among alkyl groups comprising from 1 to 10, and preferably from 1 to 6, carbon atoms; the alkyl groups comprising from 1 to 10 carbon atoms bearing at least one betaine function and/or a reactive function; and electron donor groups, notably O donors, S donors, NHCO donors, SCO donors, NHCS donors, and SCS donors, said electron donor groups may either bear or not one or several betaine groups, and/or a reactive function,
it being understood that when all the groups Ar1 represent a phenyl group, at least one of these phenyl groups, preferably two or even three of these groups, is (are) substituted with at least one group R₀ including an electron donor group;
**characterized in that** at least two of the Chrom1, Chrom2 and Chrom3 groups are substituted with at least one group R₀ bearing at least one betaine group.

5. Lanthanide complexes according to claim 4, **characterized in that** Chrom1, Chrom2 and Chrom3 are defined as follows:
a. either Chrom1, Chrom2 and Chrom3, either identical or different, are selected from the groups: 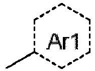 with Ar1 as defined in claim 5,
b. either Chrom1, Chrom2 and Chrom3, either identical or different, are selected from the groups: 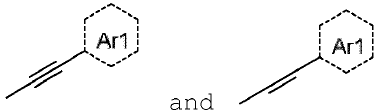
with Ar1 which represents a phenyl, thiophenyl, furanyl, pyrrolyl or imidazolyl group substituted with s groups R₀ either identical or different, s and R₀ being as defined in claim 5.

6. Lanthanide complexes according to claim 4 or 5, **characterized in that** the substituents R₀, either identical or different, are selected among:
- -L₂-Alk, -L₂-L₃-Q₁ and -L₂-L₃-Q₂;
- the NHCO donor groups selected among: -NHCO(OAlk), -NHCO(NHAlk), -NHCO(NAlk1Alk2), -NHCO(SAlk),
- the SCO donor groups selected among: -SCO(OAlk), -SCO(NHAlk), -SCO(NAlk1Alk2), -SCO(SAlk),
- the NHCS donor groups selected among: -NHCS(OAlk), -NHCS(NHAlk), -NHCS(NAlk1Alk2), and
- the SCS donor groups selected among: -SCS(OAlk), - SCS(NHAlk), -SCS(NAlk1Alk2), -SCS(SAlk),
- Alk, Alk1 and Alk2, either identical or different, are alkyl groups comprising from 1 to 10 carbon atoms, optionally substituted with at least one betaine group,
- Q₁ represents a betaine group or a branched group bearing at least two betaine groups,
- L₂ is a direct bond, -O-, -S-, -NHCO-, -SCO-, -NHCS- or -SCS-
- L₃ is a linker, and
- Q₂ is a reactive group able to allow a covalent bonding with a molecule of interest to be labeled,
it being understood that at least two of the present R₀ substituents bear at least one betaine group, so that at least two of the groups Chrom1, Chrom2 and Chrom3 are substituted with at least one R₀ group bearing at least one betaine group.

7. Lanthanide complexes according to claim 4, 5 or 6, **characterized in that** Chrom1 = Chrom2 = Chrom3.

8. Lanthanide complexes according to claim 4, 5 or 6, **characterized in that** Chrom1 = Chrom2 and are substituted with at least one R₀ group bearing at least one betaine group and Chrom3 is substituted with at least one R₀ group bearing a function -L₂-L₃-Q₂, as defined in claim 7, preferably with Q₂ which represents a group selected among -COOH, -NH₂, an acrylamide, an activated amine, an activated ester, an aldehyde, an alkyl halide, an anhydride, an aniline, an azide, an aziridine, a carboxylic acid, a diazoalkane, a haloacetamide, a halotriazine, a hydrazine, an imido ester, an isocyanate, an isothiocyanate, a maleimide, a sulfonyl halide, a thiol, a ketone, an amine, an acid halide, a hydroxysuccinimidyl ester, a succinimidyl ester, a hydroxysulfosuccinimidyl ester, an azidonitrophenyl, an azidophenyl, a 3-(2-pyridyl dithio)-propionamide, a glyoxal, a triazine, an acetylenic group, and the groups of formula: 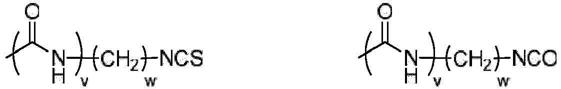 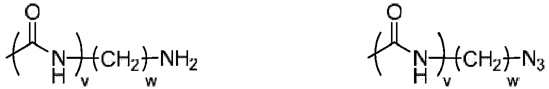 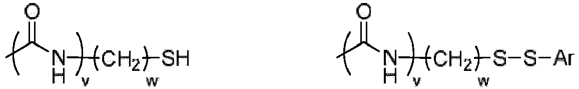 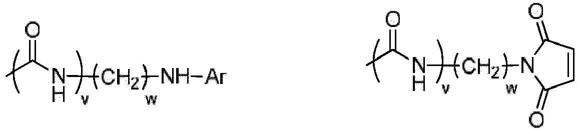 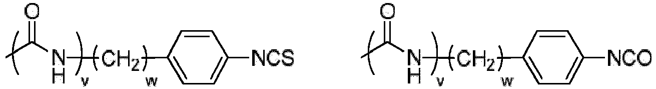 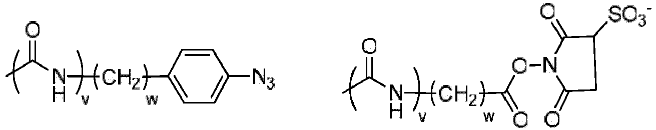 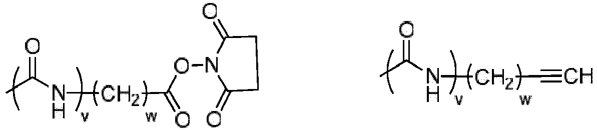 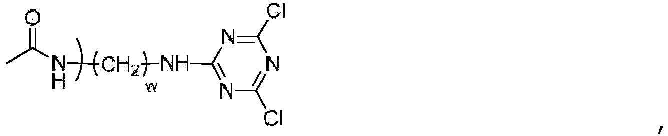
wherein w is an integer belonging to the range from 0 to 8 and v is equal to 0 or 1, and Ar is a saturated or unsaturated 5-or 6-membered heterocycle comprising from 1 to 3 heteroatoms, optionally substituted with a halogen atom;
Q₂ being preferably selected from -COOH, -NH₂, succinimidyl esters, haloacetamides, hydrazines, isocyanates and maleimides being preferred.

9. Lanthanide complexes according to one of claims 4 to 8, **characterized in that** L₁ represents a direct bond or -C≡C-, and the groups Ar₁, either identical or different, each represent a phenyl group, substituted with s R₀ groups either identical or different, with s and R₀ which are as defined in claim 5, 7, 8 or 9.

10. Lanthanide complexes according to one of claims 4 to 9, **characterized in that** in all the groups Ar1, s is equal to 1.

11. Lanthanide complexes according to one of claims 4 to 10, **characterized in that** the groups Ar1, either identical or different, each represent a phenyl group selected from the groups: 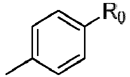 with R₀ as defined in claim 5, 7, 8 or 9.

12. Lanthanide complexes according to claim 6 or 8, **characterized in that** L₃ represents a covalent bond, an alkylene group having 1 to 12 carbon atoms, optionally comprising one or several double or triple bonds; a cycloalkylene group having 5 to 8 carbon atoms, an arylene group having 6 to 14 carbon atoms; or a sequence of one or several alkylene groups having 1 to 12 carbon atoms, cycloalkylene having 5 to 8 carbon atoms and/or arylene groups having 6 to 14 carbon atoms; said alkylene, cycloalkylene or arylene groups may or may not comprise one or several heteroatoms such as oxygen, nitrogen, sulfur, phosphorus or one or several carbamoyl or carboxamido and/or may be unsubstituted or substituted with one or several alkyl groups having 1 to 8 carbon atoms, aryl groups having 6 to 14 carbon atoms, sulfonate or oxo groups; the following linkers being preferred: 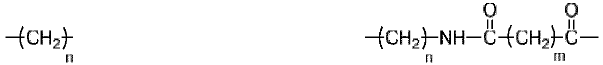 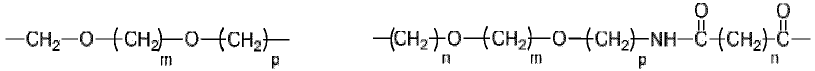 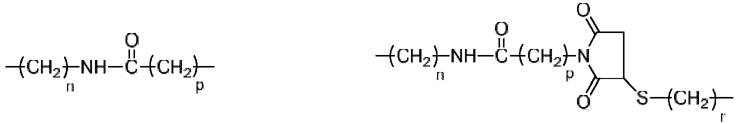 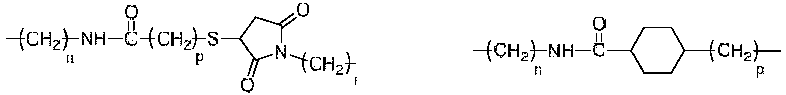 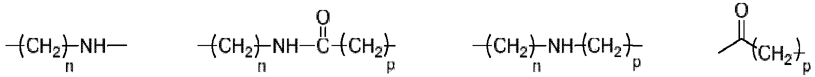 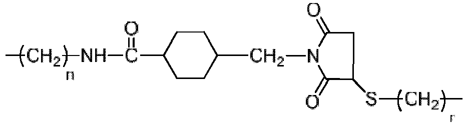
n is equal to 1, 2, 3, 4, 5 or 6,
m, p and r, either identical or different, are equal to 1, 2 or 3,

13. Lanthanide complexes according to one of claims 4 to 12, **characterized in that**:
- either Ln³⁺ = Eu³⁺ or Sm³⁺ and L₁ represents -C=C-;
- or Ln³⁺ = Eu³⁺, Sm³⁺, Tb³⁺ or Dy³⁺ and, preferably Tb³⁺ or Dy³⁺, and L₁ represents a direct bond.

14. Lanthanide complexes according to one of claims 1 to 13, **characterized in that** the betaine groups are selected from zwitterionic groups associating an aromatic iminium or ammonium cation, generally pyridinium, imidazolium, and an anionic group of the sulfonate, phosphonate or carboxylate type, preferably sulfonate, said cation and said anion being spaced apart by at least one ring member CH₂, and preferably by a bivalent alkyl chain comprising from 1 to 4, or even from 1 to 6, carbon atoms.

15. Lanthanide complexes according to one of claims 1 to 14, **characterized in that** the betaine groups are selected from: 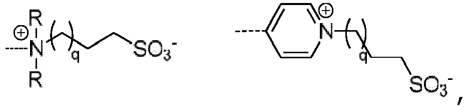 with R which represents an alkyl group having 1 to 6 carbon atoms, and preferably a methyl or ethyl, and q which is equal to 1, 2, 3, 4, 5 or 6, and preferably which is equal to 1 or 2, the group -N(CH₃)₂⁺-(CH₂)₃-SO₃⁻ being preferred.

16. Lanthanide complexes according to claims 1 to 15, **characterized in that** the betaines are water-solubilizing groups ensuring the solubilization of the complexes in aqueous medium.

17. Chelating agent of formula (II): 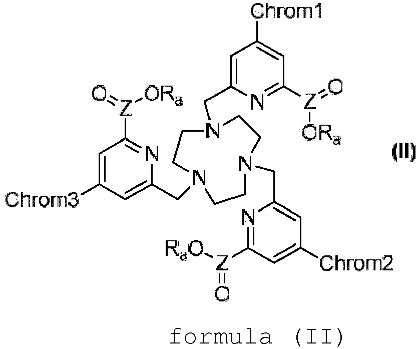 with Ra which is a protective group of acid functions such as an alkyl, of the methyl type, and Z, Chrom1, Chrom2 and Chrom3, either identical or different, which are as defined in claims 4 to 11, 12 or 14.
